# EUROPEAN PATENT SPECIFICATION

(11) **EP 0 892 069 B1**
(45) Date of publication and mention of the grant of the patent: **28.07.2010**
(21) Application number: 98111696.5
(22) Date of filing: 25.06.1998
(51) Int. Cl.: C12Q 1/68

(54) **A method for determinig the histocompatibility locus antigen class II**
Verfahren zur Bestimmung des Histocompatibiltätsantigenlokus Klasse II
Méthode de determination de locus d'antigene d' histocompatibilite classe II

(30) Priority: 26.06.1997 EP 97110438
(43) Date of publication of application: 20.01.1999
(60) Divisional application: 10152068.2
(73) Proprietor: BIO-Rad Medical Diagnostics GmbH, 63303 Dreieich (DE)
(72) Inventor: Blasczyk, Rainer, Dr.med., 14109 Berlin (DE)
(74) Representative: Keller, Günter

(56) References cited:
- EP-A- 0 443 748
- EP-A- 0 459 532
- WO-A-92/15711
- PETERSDORF E. W. ET AL.,: "Polymorphism of HLA-DRw52-associated DRB1 genes as defined by sequence-specific oligonucleotide probe hybridization and sequencing" TISSUE ANTIGENS, vol. 38, no. 4, - October 1991 pages 169-177, XP002047699

## Description

The present invention relates to methods and materials for determining the HLA Class II type of a subject, wherein group-specific sequences are used to design primer molecules which may be used in amplification protocols which accurately identify the HLA group(s) and/or allele(s) carried by the subject.

The Human Leukocyte Antigen ("HLA") Class II complex comprises the genes coding for HLA-DR, HLA-DQ and HLA-DP. The complex comprises several gene loci among which the following are especially to be mentioned: DRB1, DRB2, DRB3, DRB4, DRB5, DRB6, DRB7, DRB8, DRB9, DQA1, DQA2, DQB1, DQB2, DPA1, DPA2, DPB1, DPB2.

The HLA Class II genes are highly polymorphic among individuals. This variability is of particular relevance when tissue or organ transplantation between a donor and a host is contemplated. The histocompatibility antigens of donor and host should be as similar as possible to avoid both immune rejection of the transplanted tissue as well as graft-versus-host disease. It is therefore important to accurately identify the HLA types of donor and host. In view of the exigencies implicit in tissue transplantation, it is desirable that the typing be accomplished as efficiently as possible.

Methods for determining alleles of HLA in a patient sample have been heavily investigated because of the functional importance of these genes in transplant tissue matching and autoimmune diseases. The first tests developed used immunological methods to identify epitopes expressed by various HLA loci. These tests (*e*.*g*., the complement-dependent cytotoxicity assay described in Terasaki and McClelland, Nature, 204:998, (1964)) identified broad serological specificities but were not capable of distinguishing between allelic members of a group, and sometimes misidentified groups altogether. Unfortunately, even the most accurate of such low resolution assays cannot detect and distinguish all functionally significant transplant antigens (Anasetti et al. Hum. Immunol., 29:70 (1990)).

High resolution tests performed at the nucleic acid level which distinguish among alleles of each group have become the focus of recent research. Current methods of high resolution typing include the following.

The Sequence Specific Oligonucleotide Probes ("SSOP") technique, as described in United States Patent No. 5,451,512 assigned to Hoffmann-La Roche, Inc., uses a reverse dot blot format, wherein HLA-A probes are immobilized on a membrane, and the labelled target (patient sample) DNA is hybridized to the membrane-bound probe (as described in Saiki et al., 1989, Proc. Natl. Acad. Sci. 86:6230-6234). The pattern of hybridization to the probes on the dot-blot gives information regarding the HLA type of the individual. However, because hybridization is inherently not sufficiently specific to rule out minor differences in sequence between probe and patient sample, there is a possibility that the patient sample may contain an allelic variant which is not accounted for.

Another nucleic acid-based test is the Amplification Refractory Mutation System (ARMS) as described in the "HLA Class I SSP ARMS-PCR Typing Kit" Reference Manual, June 1995 edition, published by the Imperial Cancer Research Fund. This assay is based on the need for complementarity (matching) between the 3' end of an amplification primer and a target DNA sequence. Absent such matching, the primer will not function properly and no fragment will be amplified. Sequence information is deduced by determining, for various pairs of primers acting on target DNA from a patient sample, whether or not a fragment is successfully amplified. The accuracy of the technique is limited by the number of primer pairs tested and by the possibility that allelic variations exist in regions of DNA which lie between the primers.

In order to overcome the foregoing shortcomings, it has been proposed that typing be accomplished by direct DNA sequencing (Santamaria et al., "HLA Class II typing: direct sequencing of DRB, DQB, and DQA genes", Hum. Immunol. 33, 69-81 (1992); Santamaria et al., "HLA Class I Sequence-Based Typing" Hum. Immunol. 37, 39-50 (1993); WO 9219771; US Pat. 5,424,184). However, while direct sequencing of a patient's Class II HLA locus may conceptually be the most accurate, such sequencing may require a timeframe unsuitable for clinical practice. The success of direct sequencing methods may be expected to rely upon the design of efficient protocols and relevant primer sequences.

Prior to the present invention, direct sequencing protocols have exhibited a number of disadvantages. For example, the method of Santamaria et al., *supra,* fails to provide sufficient information because it focuses on cDNA (exon) sequences which, in view of exon sequence diversity, offer a very limited selection of conserved primer hybridization sites. In addition, because the Santamaria sequencing primers hybridize within an exon, they do not provide information for DNA sequence upstream of the primer which is potentially decisive for distinguishing among alleles. Further, the sites disclosed were determined before the recent discovery of dozens of more alleles that now need to be considered in identifying HLA type.

Intron sequences could provide the preferred hybridization sites for amplification and sequencing primers for the HLA Class II genes because they may provide the DNA sequence of the full exon.

The existence of introns, interrupting the coding regions of nuclear genes, is a typical feature of genomic DNA in complex eukaryotes. Introns are usually much longer than exons and are responsible for the major part of structural genes. In the present invention especially the sequences of intron 1 and intron 2 which are located at the 5'-end and 3'-end, respectively, of exon 2 are especially important.

A number of researchers have made limited use of intron based oligonucleotides for limited aspects of HLA Class I typing.

The European patent application EP-A-90.309107.2 of GENETYPE AG discloses an intron sequence analysis method for detection of adjacent and remote locus alleles as haplotypes. According to this method genomic DNA is amplified with a primer pair that spans a non-coding region sequence (intron). The used primer pair defines a DNA sequence which is in genetic linkage with an allele to be detected.

Blasczyk et al. (Tissue Antigens 1996: 47: 102-110) used exon based amplification primers to determine group specificity of HLA Class I. After amplification, universal sequencing primers located in intron 2 were used to sequence the amplified fragment. The paper does not disclose any intron sequence motifs from intron 1 or 3 or the 5' untranslated region. Cereb et al. (Tissue Antigens 1995: 45:1-11), undertook the identification of intron sequences useful for locus-specific amplification primer sets for all Class I genes. These primer sets were designed to amplify all alleles of the same locus. No group specific amplification primers were sought or reported. Further, amplified fragments were characterized by SSOP and not by direct sequencing.

Li W.-H. et al. [Fundamentals of molecular evolution, Sunderland, MA; Sinauer Associates (1991)] has speculated based on the information obtained from other genes that HLA Class II introns would have even more variation than the exons because of the lack of selective pressure acting on them. Since there is only very little phylogenetic relationship between HLA Class I and Class II genes it is not possible to predict whether any structural features found in the Class I genes may also be present in Class II genes.

In the course of the present invention it has been surprisingly found that the histocompatibility locus antigen class II can be advantageously determined by using a group-specific primer or primer mixture located within intron 1 and another primer located in intron 2, preferably close to the 5'-end of intron 2, whereby this antisense primer should be highly conserved and preferably group-specific, in order to allow a high resolution typing of the HLA Class II genes.

It should be stressed that the present invention makes use of group-specific motifs within the DRB introns. Those group-specific HLB-DRB sequence motifs are within the noncoding regions and can suitably used in order to amplify genomic DNA in a group-specific manner whereby the amplified DNA can be used for further investigations, preferably for sequencing.

The present invention relates to methods for high-resolution, nucleic acid-based typing of the classical HLA Class II antigen DR. It is based, in part, on the discovery of group-specific sequence motifs, derived from the analysis of numerous patient samples, which include sequences of intron 1. Such sequence motifs may be used to design amplification primers which may be used to identify the HLA group or type of a subject. The invention is also based, in part, on the determination of numerous allele-specific sequences which may be used to confirm the precise allelic type of a subject.

In the course of the present invention the remarkably conserved diversity of the HLA Class II introns which is lineage-specific rather than allele-specific has been discovered. The systematic serology-related diversity of HLA Class II intron sequences forms the basis of the present invention. This feature of HLA Class II introns has been proven to be extremely beneficial for setting up amplification strategies for template preparation.

It is a very important aspect of the present invention that by using a pair of primers several alleles can be amplified. The term "group" refers to such groups which can be distinguished by serological methods. In the present invention the groups DR1, DR2, DR3 and DR4-DR18 are of special importance. Usually in the serology of HLA groups so-called main antigens are to be distinguished from so-called split antigens. The later discovered split antigens carry group-specific epitopes and by using the split epitopes a main antigen can be subdivided in two split antigens. It should be noted that the main antigens are designated as DR1-DR10 whereby the split antigens are designated by DR11-DR18. The following relationship between the antigens should be mentioned:
DR5 can be subdivided in groups DR11 and DR12;
DR6 can be subdivided in groups DR13 and DR14;
DR2 can be subdivided in groups DR15 and DR16;
DR3 can be subdivided in groups DR17 and DR18.

For the main antigens DR1, DR4, DR7, DR8, DR9 and DR10 up to now no split antigens are known.

Each of the above-mentioned groups can be distinguished from each other by serological methods. With the help of molecular genetic methods, however, it is possible to identify a plurality of alleles for each group which have the same phenotype corresponding to the same serological appearance. The group DR11, for example, can be differentiated to presently about 30 alleles which show the same serological appearance.

Considered on the molecular level a DR antigen consists of two polypeptide chains the so-called α- and β-chain. The polymorphic form of the DR antigens is exclusively located in the β1 domain of the β-chain. This β1 domain is encoded by exon 2 of the in total 6 exons encoding the β-chain. The α-chain is not polymorph. The gene coding for the α-chain is the DRA gene and the β-chain is encoded by the gene DRB. The single genes coding for DRB have numbers and are presently designated as DRB1-DRB9. Since the genes DRB2, 6, 7, 8 and 9 are not expressed pseudogenes only the genes DRB1, DRB3, DRB4 and DRB5 are expressed. Each polypeptide chain of each of those DRB genes is associated with the same α-chain encoded by the DRA gene.

A heterodimer of polypeptide chains of DRA and DRB1 shows the serologically distinguishable specificities DR1-DR18. A heterodimer of the polypeptide chains of DRA and DRB3 shows the serologically distinguishable specificity DR52. A heterodimer of polypeptide chains DRA and DRB4 shows the serologically distinguishable specificity DR53 and a heterodimer consisting of the polypeptide chains of DRA and DRB5 shows the serologically distinguishable specificity DR51. The antigens DR51, DR52 and DR53 are separate DR molecules on the surface of the cells. Those molecules are only present when certain DR1-DR18 molecules are also present. For example the antigen DR51 is only present if also DR15 or DR16 are present. DR52 is only present if DR3, 11, 12, 13, or 14 are present and DR53 is only present if DR4, 7 or 9 are also present.

By using the term "associated group" it is to be understood that for example group DR52 comprises also the antigens DR3, DR11, DR12, DR13 and DR14.

The sequence database of HLA-DRB genes is mainly derived from mRNA analysis or has been concentrated on the polymorphic second exon. According to the present invention the structural features of the intron sequences of the HLA-DRB 1, 3, 4 and 5 genes are used.

A sequence compilation of HLA-DRB 1, 3, 4 and 5 of the 3' 500 bp adjacent to the exon 2 of the 8500 bp first intron and a fragment of 300 bp at the 5'end of intron 2 is disclosed.

The sequences used in the present invention were derived from well defined cell lines and PCR typed clinical samples. They represent all serologically defined groups and their most frequent subtypes. Beside multiple gene-specific sequence motifs, the HLA-DRB intron sequences exhibit a remarkably conserved diversity which is lineage-specific rather than allele-specific. The present invention allows therefore further insights into the genetic relationship between different alleles of HLA Class II genes and opens up the possibility for new PCR-based typing strategies using intron located amplification primers. The conserved intronic diversity will provide the comfort to establish typing systems which promise not to demand regular updates when new alleles are discovered.

The present invention provides substantially purified nucleic acids which are capable of selectively hybridizing with group specific sequence motifs in untranslated regions of HLA-D and especially the HLA-DRB1,3,4 and 5 genes. Such nucleic acids, which may be comprised in a kit, may be used, alone or in conjunction with exon-based primers, to determine the group specificity of said alleles contained in a patient sample and to identify the specific alleles present.

In particular embodiments, the present invention provides methods of ascertaining the HLA Class II type of a subject which comprise performing a first amplification reaction which identifies the group type of the subject, and a second amplification reaction which produces allele-specific nucleic acids for sequencing.

In the present invention the following definitions are used:

"Allele" means one of the alternative forms of the gene in question;

"Amplification" means the process of increasing the relative abundance of one or more specific genes or gene fragments in a reaction mixture with respect to the other genes. A method of amplification which is well known by those skilled in the art is the polymerase chain reaction (PCR) as described in United States Patents Nos. 4,683,194, 4,683,195 and 4,683,202, which are incorporated herein by reference. The PCR process involves the use of pairs of primers, one for each complementary strand of the duplex DNA (wherein the coding strand is referred to as the "sense strand" and its complementary strand is referred to as the "anti-sense strand"), that will hybridize at a site located near a region of interest in a gene. Chain extension polymerization (without a chain terminating nucleotide) is then carried out in repetitive cycles to increase the number of copies of the region of interest many times. The amplified oligonucleotides are then separated from the reaction mixture and used as the starting sample for the sequencing reaction. Gelfand et al. have described a thermostable enzyme, "*Taq* polymerase," derived from the organism *Thermus aquaticus,* which is useful in this amplification process (see United States Patent Nos. 5,352,600 and 5,079,352 which are incorporated herein by reference);

"Group" as used herein, refers to a subset of alleles of one locus, all of which share sequence features which distinguish them from other groups. For example, serological group reactivity (in a lymphocytotoxicity assay) is the conventional basis for nomenclature of HLA alleles. The first two digits of an allele refer to the serological group; for example, the designation DRB1^{*}0101, DRB1^{*}0102, DRB1^{*}0103 all are members of the DR1 group. Further, typically the nomenclature refers to the serological split group;

Since only the DRB genes are polymorphic there are only here different alleles. Those alleles are named for example for the group DR11: DRB1^{*}1101, 1102, 1103 ... up to presently DRB1^{*}1130. The first two digits refer to the serologically distinguishable group which corresponds in most cases to the split antigen. The third and fourth digit describe the subtype which can, however, be distinguished only by molecular genetic methods and means the relevant allele. It should be mentioned that there are also a fifth, sixth and seventh digit whereby the fifth digit describes mutations in the exons which do not result in a change of the amino acid sequence (e.g. DRB1^{*}11011 or DRB1^{*}11012). The sixth and seventh digit of the name of the allele describe variations in the non-coding regions of the DRB gene, namely the flanking regions at the 5'- and/or 3'-region and the introns.

"Group-specific sequence motif" means a generally short, 1-25 nucleotide ("nt") sequence of nucleic acid which is found only in one or a few groups. Where a motif is shared by several groups in one region of the HLA locus, group-specific sequence motifs in other regions of the locus may serve as group-distinguishing features. The motif may share one or more nucleotides with the consensus sequence for the region;
"Haplotype" means the allele present on one chromosome;
"Heterozygote" means the presence of at least two different alleles of a gene;
"Homozygote" means the presence of a single species of allele of a gene;
"Locus" means a gene, such as HLA-DRB1 or HLA-DRB3;
"Locus specific" means an event or thing associated with only one locus;
"Patient sample" means a sample collected from a patient in need of HLA typing which contains a sufficient amount and quality of nucleic acid (preferably DNA) for the performance of an amplification reaction. A nonlimiting example of a suitable source is peripheral blood lymphocytes, tissue (including cell cultures derived therefrom), mucosal scrapes, spleen and bone marrow;
"Primer" means a polynucleotide generally of 5-50, preferably 12-28, nucleotides length which can serve to initiate a chain extension reaction;
"Sequencing" or "DNA sequencing" means the determination of the order of nucleotides in at least a part of a gene. A well known method of sequencing is the "chain termination" method first described by Sanger et al., Proc. Nat'l Acad. Sci. (USA) 74(12): 5463-5467 (1977) (recently elaborated in EP-B1-655506, and Sequenase 2.0 product literature (Amersham Life Sciences, Cleveland) incorporated herein by reference). Basically, in this process, DNA to be sequenced is isolated, rendered single stranded, and placed into four vessels. In each vessel are the necessary components to replicate the DNA strand, which include a template-dependant DNA polymerase, a short primer molecule complementary to a known region of the DNA to be sequenced, and individual nucleotide triphosphates in a buffer conducive to hybridization between the primer and the DNA to be sequenced and chain extension of the hybridized primer. In a third series of nonlimited embodiments the group-specific primers may be used to amplify a fragment which is then identified by a sequence-specific oligonucleotide probe. In addition, each vessel contains a small quantity of one type of optionally detectably labeled dideoxynuclectide triphosphate, *e*.*g*., dideoxyadenosine triphosphate ("ddA"), dideoxyguanosine triphosphate ("ddG"), dideoxycytosine triphosphate ("ddC"), or dideoxythymidine triphosphate ("ddT"). In each vessel, each piece of the isolated DNA is hybridized with a primer. The primers are then extended, one base at a time to form a new nucleic acid polymer complementary to the isolated pieces of DNA. When a dideoxynucleotide is incorporated into the extending polymer, this terminates the polymer strand and prevents it from being further extended. Accordingly, in each vessel, a set of extended polymers of specific lengths are formed which are indicative of the positions of the nucleotide corresponding to the dideoxynucleic acid in that vessel. These sets of polymers are then evaluated using gel electrophoresis to determine the sequence.
"Specific hybridization" means hybridization of one strand of a nucleic acid to its complement.
"Target sequence" means the preferred site for specific hybridization of a primer; and
"Untranslated region" refers to a portion of an HLA locus which may be part of the mature RNA, but are not translated into protein. Examples of untranslated regions are the 5' and 3' flanking regions and intron sequences. For example, the 5' flanking region is neither transcribed nor translated, and intron sequences are transcribed but not translated.

### DESCRIPTION OF THE FIGURES

FIGURE 1 A is an illustration of the principle for an HLA Class II sequencing strategy. Group-specific primers are used for PCR amplification, and group-overlapping primers located in intron 1 near the 2nd exon and at the 3'-end of exon 2 are used for sequencing.
FIGURE 1 B shows schematically the location of the primers whereby the primers are located in intron 1 near exon 2 and the other primers are located at the 5'-end of intron 2.
FIGURE 2 A-E shows an alignment of the 486 bp of intron 1 of HLA-DRB1,3,4 and 5 genes upstream of exon 2 compared to the HLA-DRB consensus sequence. Numbers above the seqences refer to the nucleotide position within the non-coding region.
Dashes indicate identity with the consensus, asterix indicate deletions in the sequence. The 5'-end of the second exon is marked as a grey area.
FIGURE 2 G-J shows an alignment of intron 2, which is located adjacent to the 3'-end of exon 2. From FIGURE 2 G-J it can be seen that there is a group-specific polymorphism. The disclosure of FIGURE 2 G-J can be used to deduce primer sequences. With the help of those new sequences and with suitable primers located within intron 1 the whole exon 2 can be amplified by PCR. The advantage of using primers within intron 2 is that the whole exon 2 can be used for further tests, e.g. sequencing.
FIGURE 3 A shows the sequence and the localization of the PCR primers for the group-specific amplification of different gene loci, especially HLA-DRB 1, 3, 4 and 5. In Figure 3 B the primers for sequencing are shown.

The names of the sequencing primers show for which group they can be used. For example, sequencing primer DR2 can be used for sequencing the DR2 group. The sequencing primer DR3, 5, 6, 8, 10, B4 can be used with the groups DR3, DR5 (DR11 and DR12), DR6 (DR13 and DR14), DR8, DR10 of the DRB1 gene and for DR53 (the polymorphic gene coding therefore is named DRB4). The sequencing primer DRB3.2 is exclusively relevant for the gene DRB3 and has been designated DRB3.2 in order to distinguish this sequencing primer from the amplification primer DRB3.1.

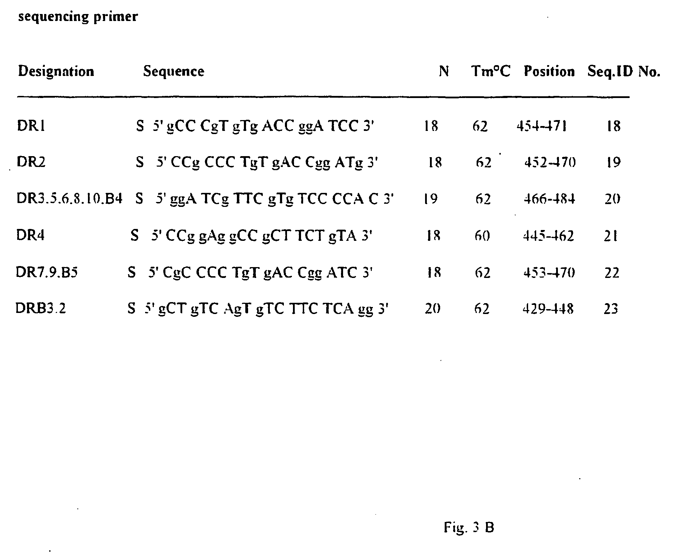

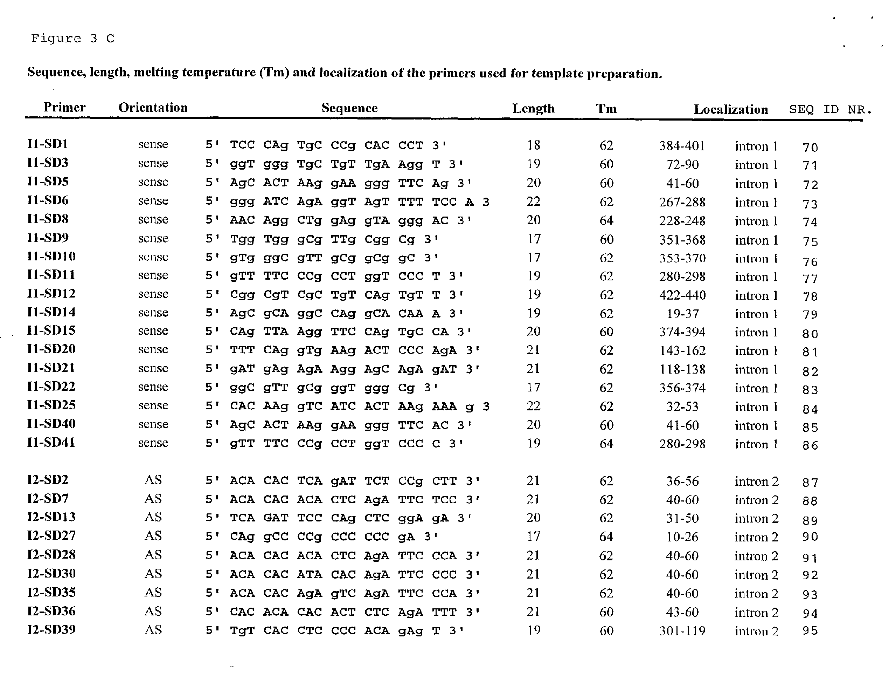

FIGURE 3 C shows amplification primers which are located within intron 1 and on the other end within intron 2. When the primers shown in FIGURE 3 C are used the sequencing primers of Figure 3 B can be used.

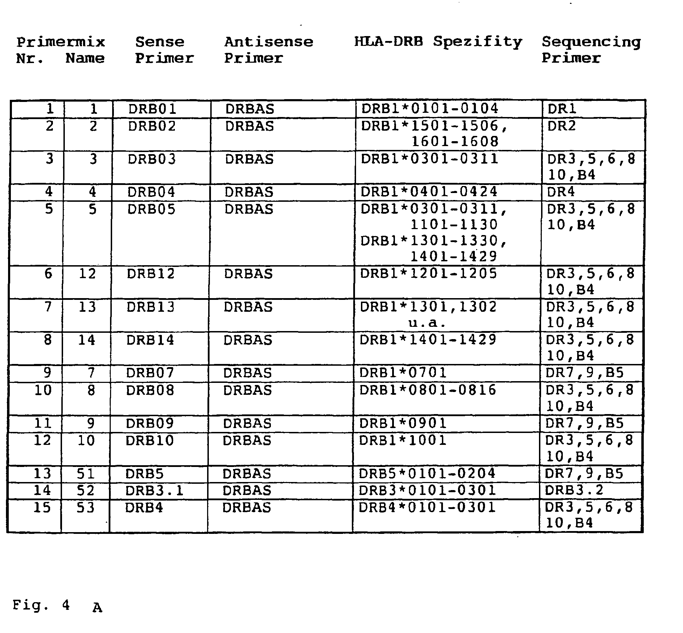

FIGURE 4 A shows the specificity of the primer mixtures for the production of templates. The selection of the mixture of primers for sequencing was done on the basis of a low discriminating pretyping. The designation of the mixture of primers was chosen according to the serologically defined groups DR1-10, DR12-14 and DR51-53.

The used primers show a clear group-specificity which relates to the serologically defined groups. This specificity is expressed in the name of the primer mix. The primer mix having the name 1, for example, can be used in order to amplify the alleles of the DRB1 gene which causes the serological specificity DR1.

A more complicated example is the primer mix having the name 5. This primer mix can be used in order to amplify alleles of the serological specifity DR5, namely the subgroup DR11. Moreover this primer mix amplifies, however, the subgroups DR3, DR13 and DR14.

The group-specificity is based on the specificity of the 5'-primer located in the first intron (for example DRB01-DRB05). All primer mixes contain the antisense primer at the 3'-end named DRBAS. This primer is located within a sequence at the end of exon 2 having no variability. This sequence is common for all DRB genes (DRB1, 3, 4 and 5). According to this invention a group-specific primer located in the 5'-end of intron 2 is used.

Since all primer mixes cover the variability of the exon 2 it is possible to determine by sequencing of the obtained PCR products the exact allele of the DRB gene.

As an alternative to the fragment length the hybridization to a sequence-specific oligonucleotide probe may be used as a criterion for the identification of an individual product of amplification. Detection of oligonucleotide hybridization can be achieved by different techniques well established in the field. This approach may be useful if two possible products have the same length but a different sequence or if the group-specific amplification is part of a typing system working by hybridization.

It has been found that at the end of intron 1 there is no universal sequence which allows the design of a sequence primer usable for all groups. This represents an essential difference with regard to the situation of HLA Class I. Therefore, group-specific sequencing primers have to be used which has, however, the advantage that the quality of sequencing is improved. The sequencing primer (as shown in Figure 3b) have names corresponding to the groups for which they are specific.

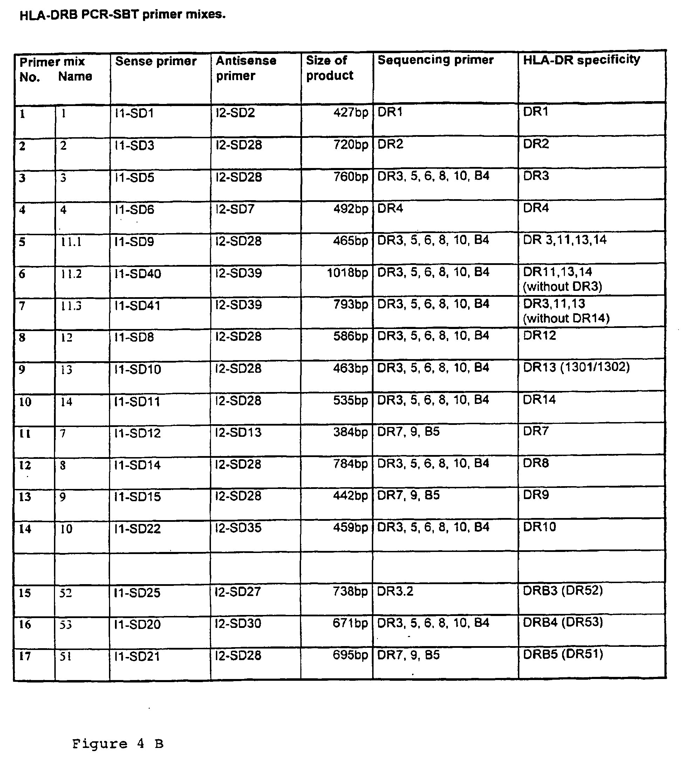

FIGURE 4 B shows the specifity of primer mixtures for the production of templates, wherein the sense primers are located within intron 1 and the antisense primers are located within intron 2.

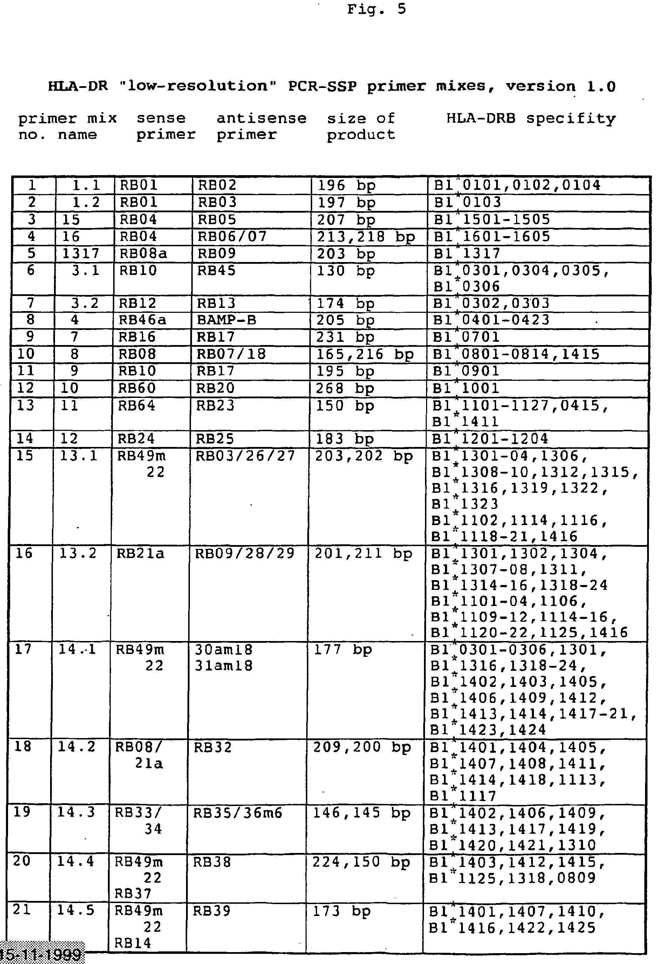

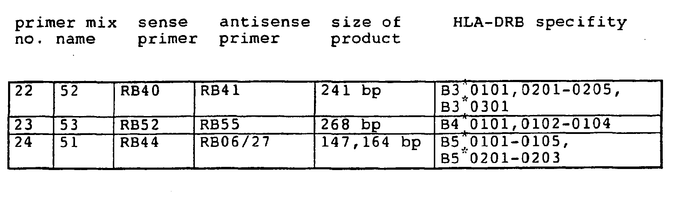

FIGURE 5 shows primer mixes which can be used for pretyping. The primers are located in exon 2.

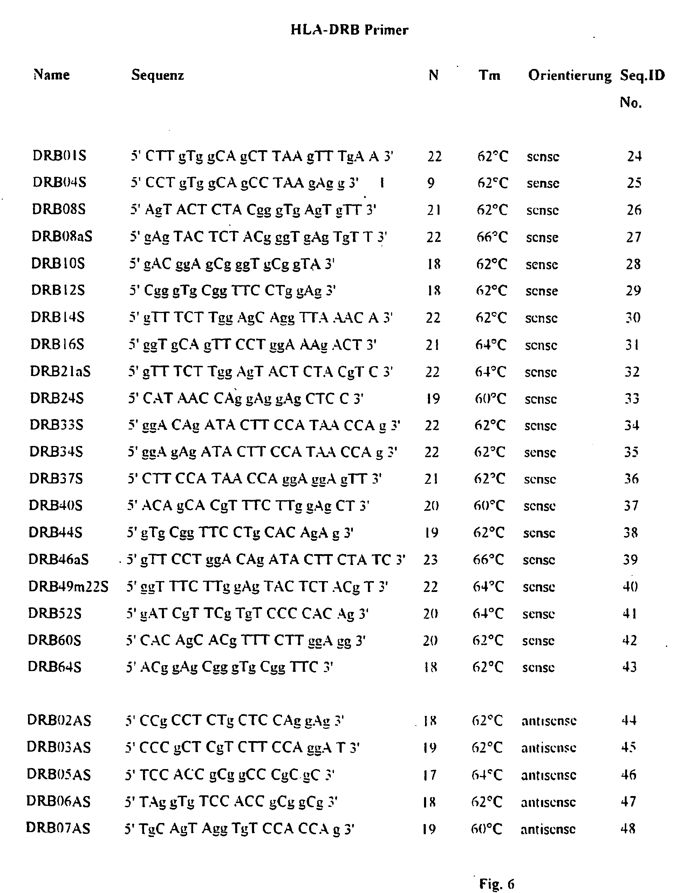

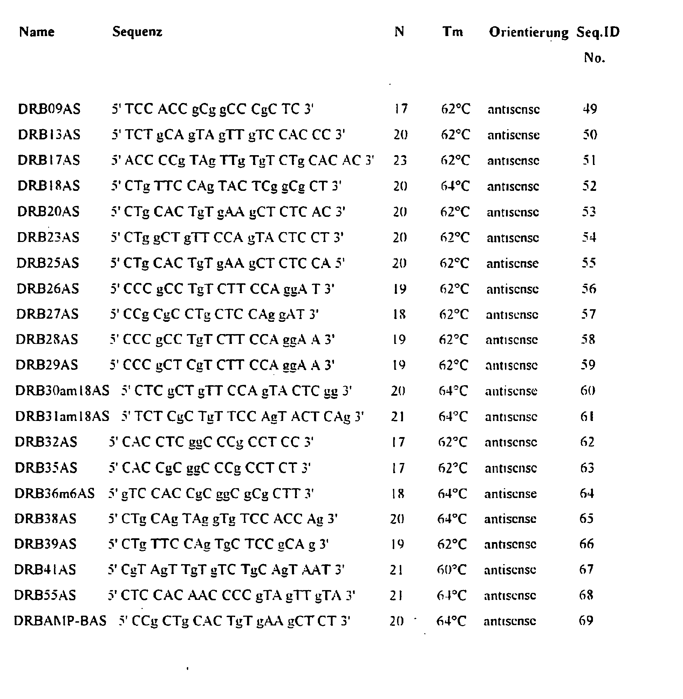

FIGURE 6 shows the nucleotide sequences of the primers mentioned in Fig. 5.

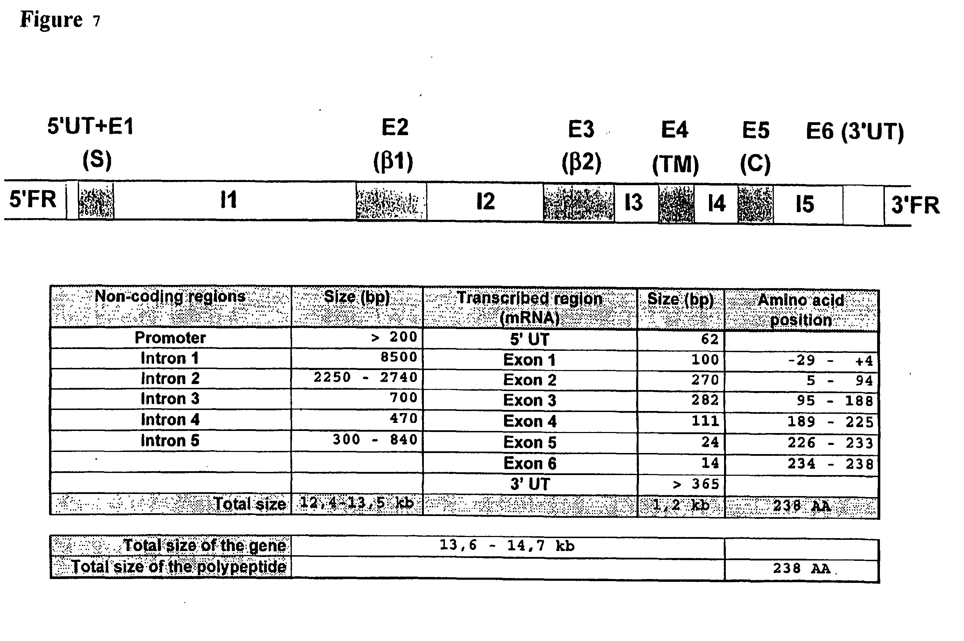

FIGURE 7 shows schematically the structure of the HLA-DRB gene. Above the sequencing of exon 2 has been described in detail. It is, however, possible to use the teaching of the present invention in order to amplify also other exons.

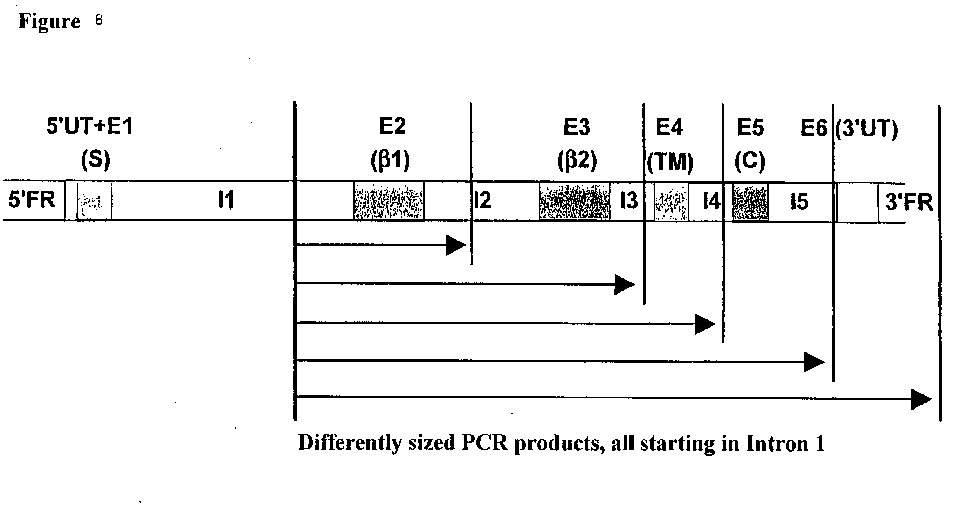

FIGURE 8 shows schematically the amplification of larger sequences. In order to achieve larger DNA fragments containing not only intron 1, exon 2 and intron 2 the antisense primer has to be located within the next intron, e.g. intron 3, 4 or 5 or the 3'-flanking region. By using the described sense primers in intron 1 and using for example an antisense primer located within intron 3 the amplificate would stretch from the 3'-end of intron 1 over exon 2, intron 2 and exon 3 up to intron 3.

### DETAILED DESCRIPTION OF THE INVENTION

The present invention relates to compositions and methods which may be used to efficiently and accurately determine the HLA Class II type of a sample derived from human and non-human primates, especially human beings, e.g. patient donors for transplantation and forensic purposes.

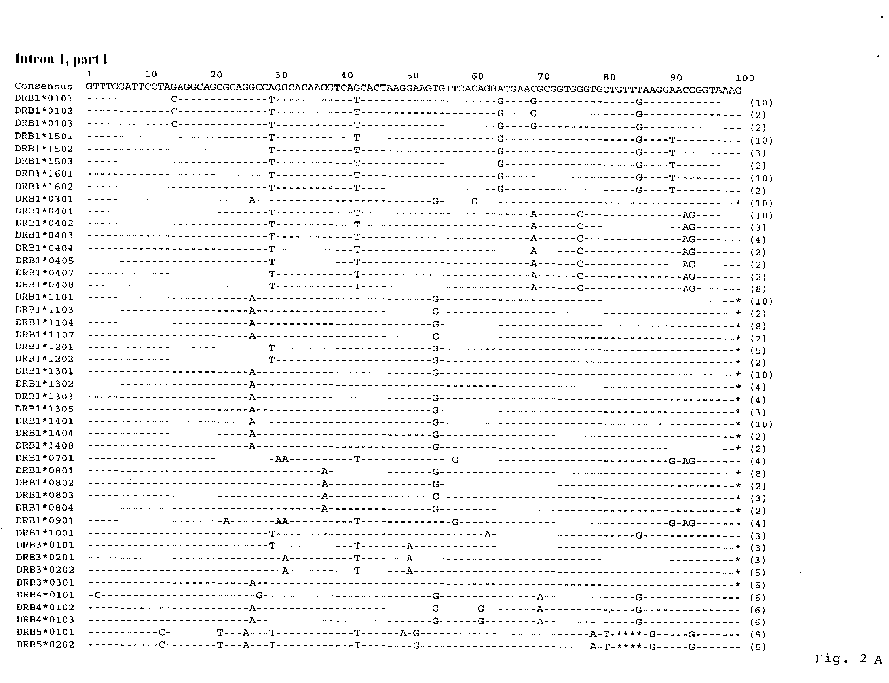

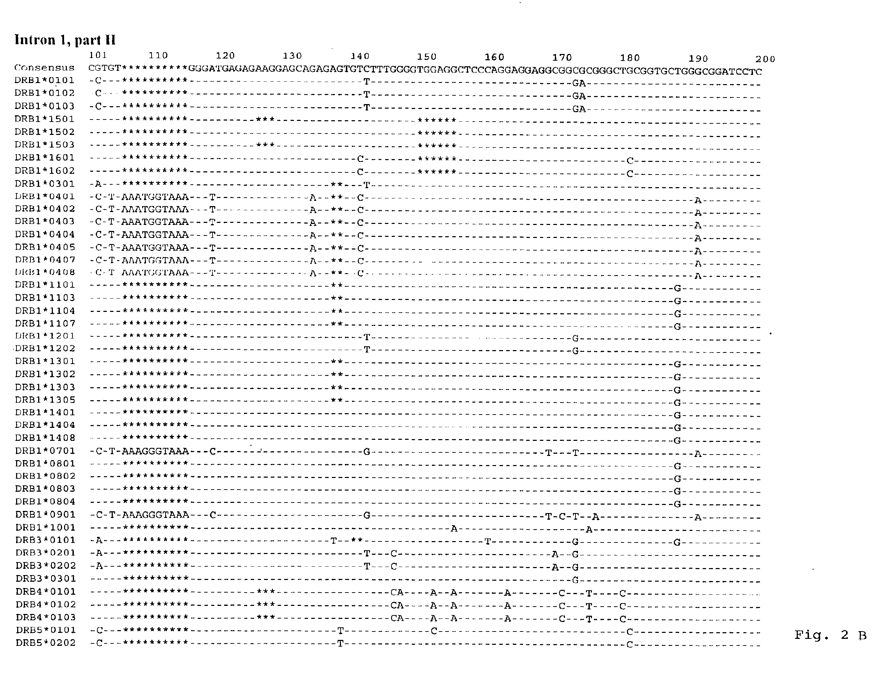

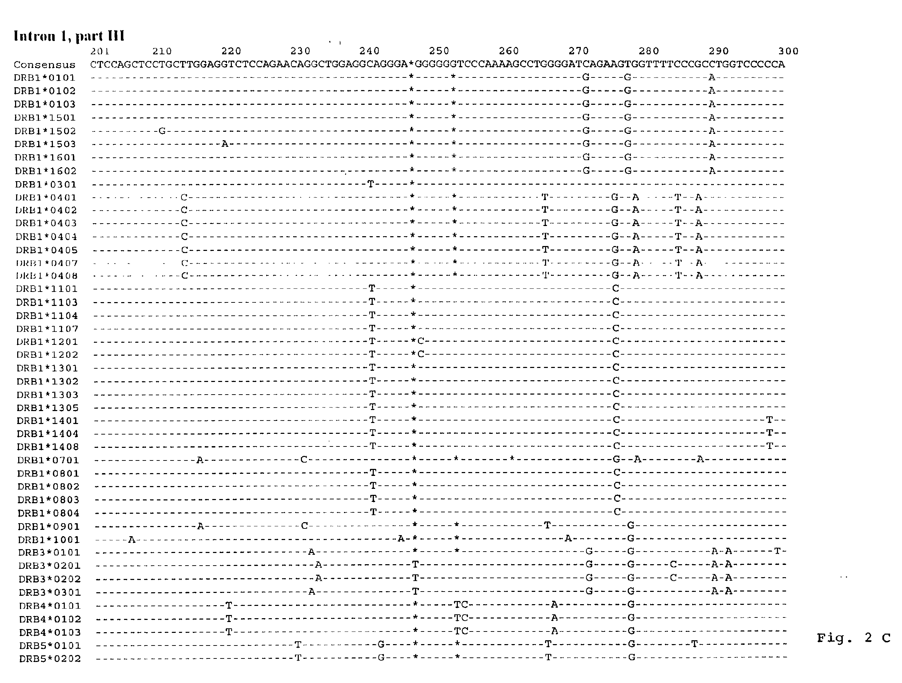

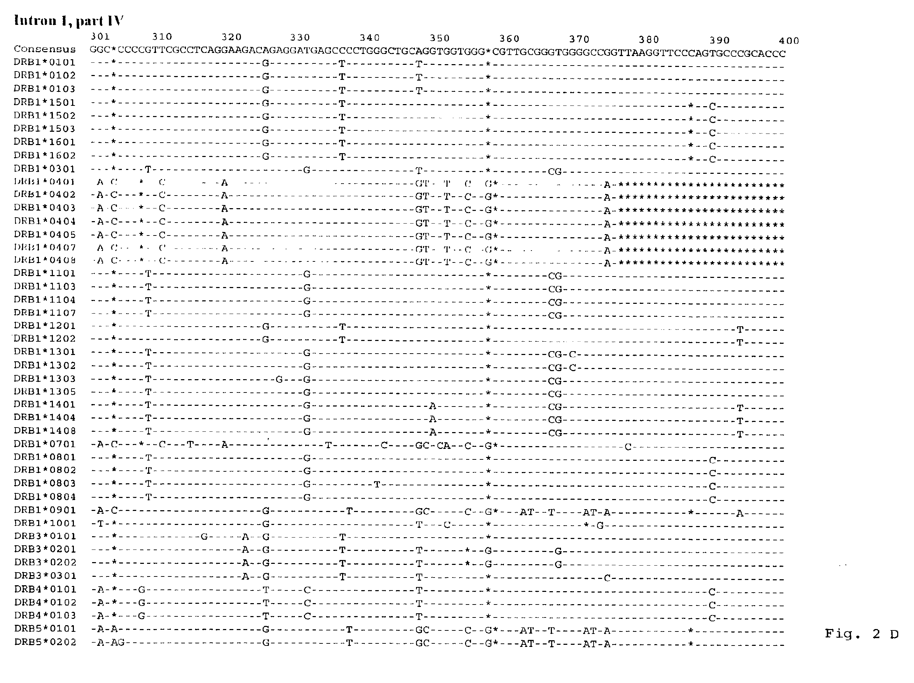

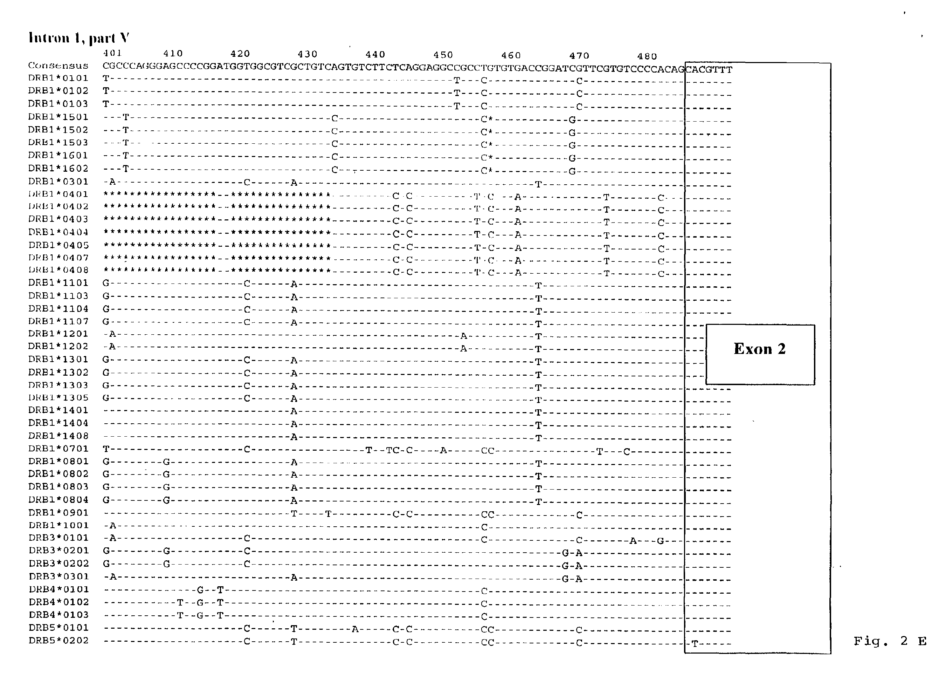

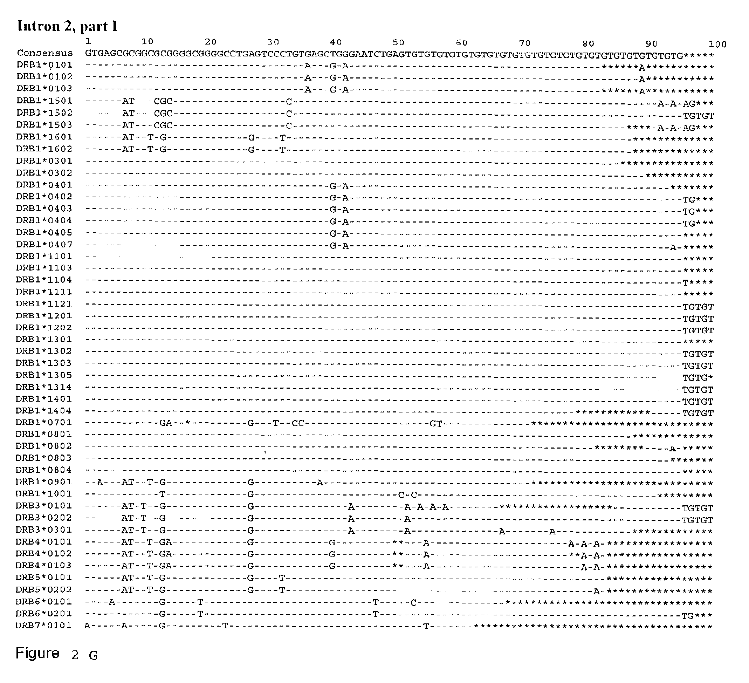

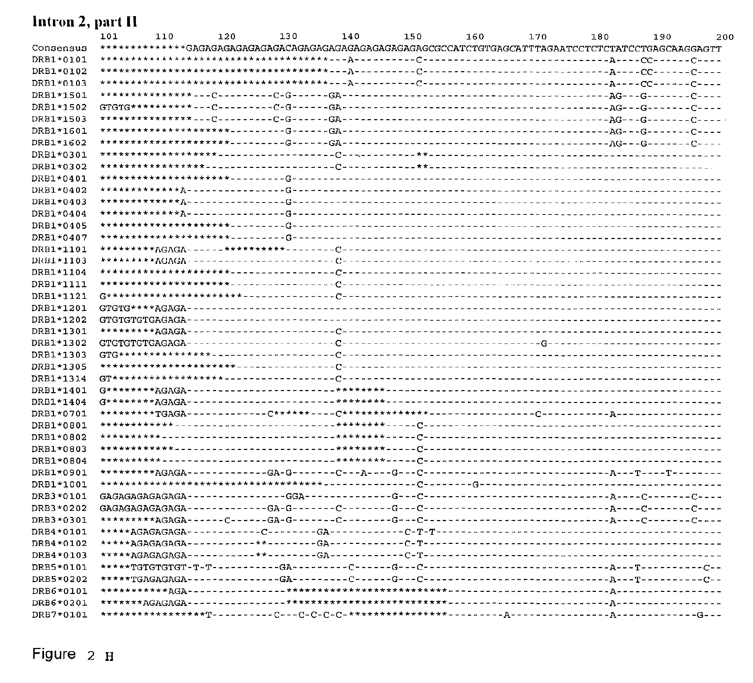

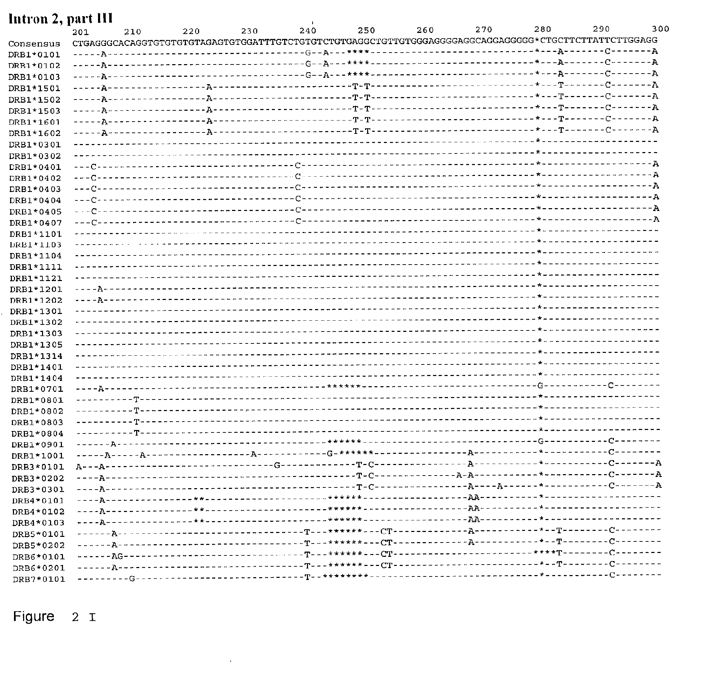

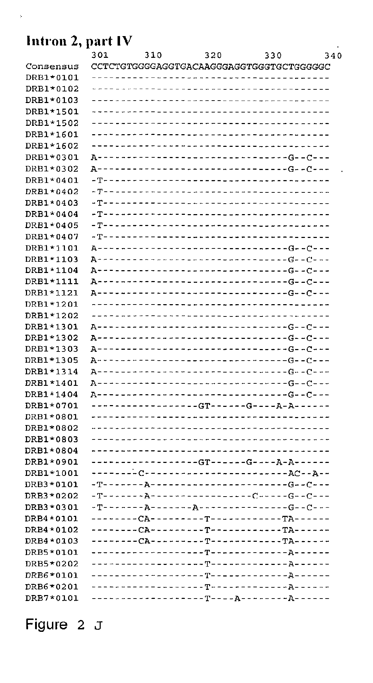

The present invention is based, in part, on the determination of group-specific sequence motifs in certain regions, especially intron regions of HLA Class II loci. These motifs may be used to design oligonucleotides which may be used as group-specific primers in nucleic acid amplification reactions. Alternatively these motifs may be used for the design of probes which are preferably group-specific. The present invention is also based, in part, on the determination of the sequences of regions of a wide variety of alleles of HLA Class II loci; such sequences may be used to distinguish one allele from another. Sequences of intron 1 of HLA-DRB genes are provided herein, and are set forth in Figure 2.

In general, the methods of the invention may be described as follows. Comparison of nucleotide sequences of an HLA locus among members of an HLA Class II group, which lie in either untranslated or exon regions, may be used to identify group-specific motif sequences. Identification of groups may be by establishing serological relationships or using phylogenetic information. Based on the group-specific motif sequences, oligonucleotide primers may be designed, synthesized, and used to amplify a portion of the HLA locus. Oligonucleotides used in this manner are referred to herein as "group-specific primers" and, in particular, as "group-specific untranslated region primers".

In preferred nonlimiting embodiments of the invention, the primers correspond to untranslated regions of the HLA Class II locus ("group-specific untranslated region primers"). Such primers may be used in pairs, wherein each member of the pair hybridizes to an untranslated region lying on either side of at least one exon. For example, but not by way of limitation, primer pairs may be oligonucleotide pairs which hybridize to group-specific motifs in the first and second introns.

The group-specific primers may be used in several different methods according to the invention. In a first series of nonlimiting embodiments, the group-specific primers may be used in a diagnostic manner to identify which allelic groups are present in a patient sample. In a second series of nonlimiting embodiments, the group-specific primers may be used to amplify sufficient amounts of a particular allelic fragment which is then subjected to direct nucleotide sequencing using suitable sequencing primers.

According to the first series of embodiments, the present invention provides for a method of determining the HLA Class II group type which is part of the HLA-DRB1, HLA-DRB3, HLA-DRB4 or HLA-DRB5 locus of a subject comprising
(i) combining a group-specific primer pair with a target DNA sample from the subject under conditions such that primer-based amplification of the target DNA may occur; and
(ii) determining whether a nucleic acid product is produced by the amplification; wherein the ability of a primer pair to produce a nucleic acid product is associated with a particular HLA group type. The group-specific primers are group-specific untranslated region primers, wherein the sense primer is located in intron 1 and the antisense primer is located in intron 2.

The first method may be used to provide useful diagnostic information. For example, group type determination may serve as a first level of comparison for a histocompatibility analysis, even without identification of the specific allele(s) involved. For example, if a potential donor and host are being evaluated for tissue transplantation, and, if it is found that their group types do not match, no further comparison may be necessary. If, alternatively, their types do match, further analysis, for example by direct sequencing, may be desirable.

According to the second series of embodiments, the present invention provides for a method of determining the HLA Class II allelic type of a subject comprising
(i) combining a group-specific oligonucleotide primer pair with a target DNA sample from the subject under conditions such that primer-based amplification of the target DNA may occur; wherein the sense primer is located in intron 1 and the antisense primer is located in intron 2;
(ii) collecting the nucleic acid product of the amplification; and
(iii) determining the nucleic acid sequence of the product.

The group-specific primer pair used may be determined based on the group type of the subject, as determined using the first method, described above. In preferred embodiments of the invention, group-specific untranslated region primers which span a region of the HLA locus containing allele-specific sequence may be utilized. If a subject is heterozygous, separate amplification reactions are performed for each group identified. Sequencing may be performed using group-overlapping sequencing primers which will operate for several groups.
According to the third series of embodiments the present invention provides for a method of determining the HLA Class II group type which is part of the HLA-DRB1, -DRB3, -DRB4 or -DRB5 locus, or allelic type of a subject comprising
(i) combining a group-specific oligonucleotide primer pair with a target DNA sample from the subject under conditions such that primer-based amplification of the target DNA may occur, wherein the sense primer is located in intron 1 and the antisense primer is located in intron 2;
(ii) hybridizing the nucleic acid product of the amplification with a sequence-specific oligonucleotide probe;
(iii) determining whether the oligonucleotide probe has hybridized to the amplification product; and
(iv) assigning, dependent on the specific oligonucleotide, a group or allelic type to the amplification product.

In a further preferred embodiment of the present invention it is possible to use an intron region primer pair which is specific for several loci. It is for example possible to design primer pairs which are specific for several loci preferably selected from the group consisting of DRB1, DRB3, DRB4 and/or DRB5. Alternatively it is also possible to use an intron region primer pair which is specific for a single locus.

The oligonucleotide probe which is used for the hybridization with the amplification product is preferably group-specific whereby either motifs derived from an exon or motifs derived from the corresponding intron(s) can be used.

A more detailed description of the invention follows. Most alleles of the classical HLA Class II gene loci (consisting essentially of HLA-DRB genes) can be distinguished on the basis of exon 2 only. In one non-limiting embodiment, a method of the invention takes advantage of this fact, and employs the strategy generally described above, using the example of HLA-DRB.

A genomic DNA sample is prepared from a patient sample according to well known techniques. Aliquots of the genomic DNA may then separately be reacted with a panel of group-specific exon region primer pairs, wherein the successful amplification of a DNA fragment is associated with a particular group type.

Each primer pair in the cocktail will amplify only selected allelic groups because at least one of them specifically hybridize to group specific intron sequence motifs. Between them, under suitable polymerase chain reaction (PCR) conditions, the cocktail may amplify all known HLA-DR or HLA-DRB groups, with each group specific amplification product having a different length. When reaction products are separated on an agarose gel the group(s) present in the patient sample may be identified by length.

Optionally, once the group specificity is determined, the direct sequence of alleles may be determined for precise allelic identification. A further part of the patient sample DNA may be treated under PCR conditions with a pair of primers that are specific for the previously determined group; preferably such primers are group-specific untranslated region primers, which span greater distances of the locus. If two groups were detected, then two separate reactions are performed. At completion of the second amplification, the reaction products are sequenced using an intron based "sequencing primer" which hybridizes to an intron sequence which may be conserved among alleles of several groups. Though it is theoretically possible to use a sequencing primer which is specific for the amplified group only, it is found that using a group-overlapping primer simplifies the method and the preparation of a kit. Various group-overlapping sequencing primers are specifically provided herein which hybridize, respectively, to intron sequences flanking the 5' end of exon 2 and the 3' end of intron 2.

The substantial advantage of the method of the invention is that the initial group specific amplification allows a PCR based separation of haplotypes in 95% of patient samples. The separation of the haplotypes is a major achievement of this protocol since it permits the resolution of cis/trans linkages of heterozygote sequencing results which cannot be achieved with other protocols. With the instant invention, a separation of the haplotypes may be achieved in serological heterozygous samples with the sequencing primer mixes ("PMs") using group-specific amplification corresponding to the serological families. The selection of the PMs used for sequencing depends on the amplification patterns of the preceding PCR-SSP low-resolution typing. The primers are designed to work within a single cycle protocol including, but not limited to, a PCR protocol on a Perkin Elmer System 9600, maintaining typing capacities of the laboratory. All PCR products carry sufficient sequence information for a complete subtyping. This approach is superior to a typing system using a single pair of generic primers followed by direct sequencing or SSO hybridization, even if the amplification strategy is locus-specific. The substantial advantage of Sequencing Based Typing (SBT) is the definition of the cis/trans linkage of sequence motifs. SBT after generic PCR amplification cannot define the cis/trans linkage of sequence motifs and therefore mimics oligotyping. The rapidly growing number of newly identified alleles confirms that new alleles have arisen mainly from gene conversion events which have usually taken place between different alleles of the same locus. Newly identified alleles are not characterized by new sequence motifs, but by a new combination of already existing sequence motifs. From this observation it may be concluded that the amount of alleles at each locus may theoretically represent all possible combinations of known sequence motifs. Of course, some of them will fall victim to negative selection. Nevertheless, it can be expected that still an enormous amount of alleles are yet unidentified. PCR-SSP subtyping strategies using a restricted number of oligonucleotides which do not cover all possible sequence motifs suffer from this limitation. If the cis/trans linkage of the analyzed polymorphic regions is not defined some new alleles may be mistyped as a heterozygous combination of known alleles. This has consequences with respect to SBT strategies. An unambiguous typing result of SBT after generic PCR amplification is only unambiguous with regard to the presently known HLA sequence databank.

In general, group-specific primers are desirably designed to facilitate hybridization to their intended targets. It should be taken into account that homology between different groups, and indeed between group-specific motifs, may exist. Accordingly, in preferred embodiments of the invention, a primer may be designed such that it hybridizes to its group target under relatively stringent conditions. For example, one or more mismatched residues may be engineered into the 3' domain of the molecule. Further, the primer may be designed such that it differs from any naturally occurring or consensus sequence, but rather has mismatches inserted which serve to further reduce hybridization of the primer to target DNA of a group other than the intended target group. Under certain circumstances, one or more mismatches may be introduced into the 5' end to destabilize internal hairpin loops; such changes are not generally expected to enhance the efficiency of the primer.

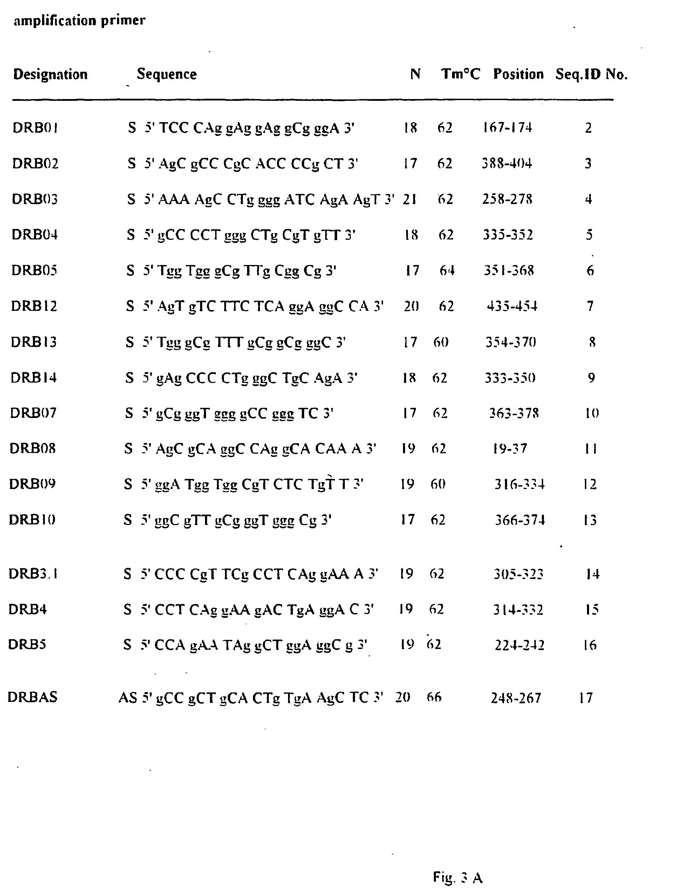

The nucleic acid sequences SEQ ID NOs: 2-16, 18-23 and 70-95 as given in Figure 3 may be comprised in group-specific untranslated region primers for HLA-DRB, which are specific for the groups as indicated in Figure 4.

In general, the foregoing group-specific primers may be modified by addition, deletion, or substitution of bases, to produce functionally equivalent primers with the substantially same specificity, that is to say, such that the group specific polymorphism(s) are not removed. Such modifications may be constrained by several parameters. First, exact matching at the 3' end is particularly important for primer extension. Preferably, at least 5 nt are complementary to target DNA. When the exactly conserved region is short, for example, less than 10 nt, it is not advisable to change the primer sequences. The primer is preferably less than 50% G or C. Also, the primers should be designed to avoid specific hybridization with pseudogenes. In the examples which follow, the melting temperature of all primers used was about 62°C to ensure uniform amplification conditions.

In a further preferred embodiment of the present invention a pretyping is performed. In the course of this pretyping it is checked which groups are present in the patient. Because of the diploid chromosomes only two groups are possible. It would be possible to use all primer mixes in parallel assays with one probe and afterwards it is checked which primer mix has resulted in an amplification product. This product can then be sequenced. In one preferred embodiment a pretyping is done by using primer mixes located within the second exon. Those primer mixes amplify group-specific only a small fragment of exon 2. These primer mixes are, however, not suitable for sequencing since they contain only a small part of the variability of the exon and by sequencing no further information is obtained. The primers are shown in Figure 5. The sequences of the primers disclosed in Figure 5 are shown in Figure 6.

In this embodiment of the present invention a pretyping is done by using the primers located in the exon and in a second step the primer mix as shown in Figure 4 is used for the preparation of the template. This is possible since there is a strict correlation between the intronic sequences and exonic sequences.

The selection of suitable universal sequencing primers is constrained by a variety of rules including the following. Sequencing primer hybridization sites must lie within the fragment amplified by the group specific amplification primers. All primers are desirably selected to provide informative sequence and not start too far upstream of useful sequence. Preferred primers hybridize to conserved sites near the exon/intron boundaries.

An important issue of direct sequencing for HLA class II genes is the generation of a specific PCR product, which is rather complicated due to the extensive sequence homologies between the different HLA class II loci including several pseudogenes. If an adequate PCR product has been generated, any sequencing chemistry should be applicable.

In the normal case, since group specific amplifications take place before sequencing, only one allele at a time is sequenced, resulting in unambiguous homozygous sequencing results. In these cases alleles are relatively easy to identify, even without software.

However, in about 5% of cases, both alleles come from the same group, but the sequence results show heterozygosity. In practice, when viewed by a fluorescence-detecting system, the sample appears as a normal sequence of bases with, sporadically, two bases at one site, each with half the peak height. This result flows from the high degree of similarity shared among all alleles of each HLA gene; sequence heterozygosity flows from base substitutions. The laborious task of determining which alleles are present in the test sequence may be simplified using computer analysis. A software program, for example GeneLibrarian (obtainable from Visible Genetics) rapidly compares the test sequence to a database which includes all possible homozygote and heterozygote combinations of the alleles. The program identifies those stored sequences that are closest matched to the test sequence. The operator can then determine which allelic pair is in the test sample. If no allelic pair shows an exact match, the software allows the operator to review the test sequence to determine if errors in base-calling or other artifacts are interfering with the analysis.

The order of sequencing reactions may be selected by the operator. Each exon of each locus may be sequenced on the sense strand or anti-sense strand. A preferred method is to obtain sequence from one strand from the exon. If the results contain ambiguities, then the amplicon is re-sequenced using the other primer for the same exon. The availability of both sequencing primers provides redundancy to ensure robust results.

In some cases, it may be advantageous to employ an equimolar mixture of 2 or more oligonucleotide species. Mixtures of oligonucleotides may be selected such that between them they will effectively prime the sequencing reactions for all alleles of the locus at the same site.

In an alternative technique, instead of using dye terminators, a dye-labelled primer may be employed. In this case, the selected sequencing primers is labelled on the 5' end with a detectable label, using phosphoramidite or NHS/dye ester techniques well known in the art. The label selected depends on the detection instrument employed. The label for use with an OpenGene System (Visible Genetics Inc., Toronto, ON) is the fluorophore Cy5.5 (Amersham Life Sciences, Cleveland OH). Fluorescein-isothio-cyanate may be used for detection with the ALF Automated Sequencer (Pharmacia, Piscataway NJ). In this method, which is well known to one skilled in the art, the sequencing reaction mixture is changed slightly to include only one ddNTP per reaction mixture. For detection of reaction products, the sample may be mixed with an equal volume of loading buffer (5% ficoll plus a coloured dye). 1.5 µl of these samples may be loaded per lane of a MicroGel electrophoresis cassette loaded in a MicroGene Blaster automated DNA sequencer (Visible Genetics Inc., Toronto). The sample may be electrophoresed and read.

Results may be displayed and analyzed with GeneObjects software. The sequence of bases may be determined, and the HLA allele to which the sequence corresponds may then be identified. This process may be performed for each locus (HLA-DRB1-5) and the results may then be reported to the patient file.

It is well known in the art that different variations of sequencing chemistry may be employed with different automated DNA sequencing instruments. Single dye instruments, such as the OpenGene System (Visible Genetics Inc., Toronto), the ALF Express (Pharmacia, Uppsala, Sweden) or the Li-Cor 4000L (Lincoln City, Nebraska) generally use dye-labeled primers. In these systems a single chain termination sequencing reaction mixture is run per lane.

Multi-dye sequencers, such as the Prism 377 (Applied Biosystems, Inc., Foster City, California) detect multiple dyes in a single lane. This technology conveniently employs dye-terminator chemistry, where the chain-terminating nucleotides are themselves labeled with fluorophores (see United States Patent No. 5,332,666, to Dupont de Nemours and Co.). In this case, the reaction products carrying four different labels may be run in a single lane.

Either single dye or multi-dye chemistry may be employed according to the present invention, along with other sequencing chemistries.

The nucleic acids described above may be comprised in a kit for use in practicing the methods of the invention. In addition to the group-specific primers and primer pairs disclosed, such kits may further comprise buffers, reagents, and enzymes such as, amplification enzymes including but not limited to, *Taq* polymerase. In specific, non-limiting embodiments, the kit may comprise group-specific exon region primers (for example, as a "cocktail" comprising a plurality of primers) as well as group-specific untranslated region primers; such primers may be contained in individual tubes. Methods of high resolution typing are detailed in the examples below, which examples are set out to exemplify the method of the invention and not to limit the scope of it in any way.

HLA-DRB genes are about 12 KB in size. About 93% of the genes are made up by non-coding sequences, whereas only 801 bp from exon 1 through exon 6 carry the coding information, representing less than 7% of the gene's nucleotides. The polymorphism of the coding region is exclusively restricted to the β1-domain encoding exon 2 which represents the preferred embodiment of the present invention.

Based on the diversity of this exon 2 216 HLA-DRB 1, 3, 4, and 5 alleles can be distinguished according to the latest WHO nomenclature report [Tissue Antigens (1997), vol. 49, p. 297-321]. This gives impressive insights in the peptide presenting capacity of the human population and it can be expected that a tremendous number of alleles will be identified in the future.

Despite profound knowledge of the different coding regions nearly nothing is known about the intervening intron sequences.

The HLA-DRB intron sequences are not characterized by random point mutations but by a highly systematic diversity, reflecting the different DRB lineages. With a few more exceptions than in HLA Class I the variability is arrested on the level of the serological diversity with almost no sequence variations between the subtypes of the same serological group.

The few intragroup variations detected in DRB1*15 (1502 and 1503), DRB1*13 (1302, 1303, 1305) and DRB1*08 (0803) as well as in DRB4 and 5 alleles are with the exceptions in DRB1*13 (nucleotides 54 and 370) and DRB4*0101 (nucleotide position 61) all unique point mutations, which cannot be explained by intronic or interlineage recombination events. These deviations do not affect the group-specific intron motifs. The differences found in DRB3 alleles are in accordance with the serology-related character of the intronic diversity, showing sequence differences between DR52a, 52b and 52c, but not between the two alleles belonging to DR52b (DRB3*0201 and 0202).

In the course of the present invention no evidence has been found that recombination events diversifying the exons do involve the intron regions. However, this cannot be completely excluded.

The striking conservation within each ancestral lineage suggests that point mutations have been negatively selected. Consequently it can be assumed that introns carry a functional relevance and that they are not as much subjected to evolutionary diversification forces as the exons. Based on the finding of the present invention it can be expected that the serology-related motifs in the non-coding regions are highly conserved, even if the intron sequences are not available from all alleles. Whatever the function is, it maintains the strictly exon-correlating variability of the intron sequences.

The finding of the present invention has substantial consequences for PCR based typing strategies. New alleles often cause ambiguous amplification patterns in exon-based PCR protocols and therefore demand a regular update of the typing system. This experience has been made in HLA Class I and II typing during the last years and will surely continue with the permanent detection of new alleles.

The intron-restricted amplification method of the present invention will probably not suffer from these limitations. Based on the now available intron data, it is assumed that new alleles will correspond to the intron sequence motifs described so far. Therefore, these motifs are selected as priming sites for group-specific PCR amplification.

### Example 1

### a) DNA samples

Genomic DNA was prepared by a standard salting-out procedure. A set of 25 well defined lymphoblastoid B-cell lines mostly from the 9th and 10th international histocompatibility workshop and 191 PCR typed clinical samples, representing all serological HLA-DRB antigens from different ethnical backgrounds and their most frequent subtypes, were analysed.

### b) PCR and sequencing primers

For sequencing of the non-coding regions, PCR-based template preparation was carried out, haplotype-specific by the use of a generic intron-located primer (19mer, 5' AgC ggA gTg gAg Agg TCT g 3' [SEQ ID No. 1], Tm 62°C) and group- or allele-specific primers in the 2nd exon. The design of the generic 5' primer relied on the very limited number of intron 1 sequences available from the EMBL databank. The resulting products carried, depending on the amplified allele, about 600 bp of the 1st intron of a single allele flanked by variable stretches of 2nd exon sequences. The PCR primers and several nested primers were applied as sequencing primers.

### c) PCR conditions

The PCR reaction mixture in a final volume of 50 µl consisted of 500 ng genomic DNA, PCR-buffer [60 mM Tris-HCL, pH 9.5; 3.5 mM MgCl₂; 15 mM (NH₄)SO₄], 200 µM of each dATP, dCTP, dGTP and dTTP and 2.0 U DNA Taq Polymerase (Amplitaq^{™}, Hoffmann-La Roche, Basel, Switzerland). For sequencing of the 1st intron biotinylated PCR products were generated using 10 pmol of the biotinylated generic 5' primer and 15 pmol of unlabeled 3' primers. PCR amplifications were carried out in a GeneAmp PCR System 9600 (Perkin Elmer Cetus Corp., Norwalk, CT). All primer mixes worked with the same PCR profile. After an initial denaturation step at 94°C for 2 min, samples were subjected to 15 two-temperature cycles, each consisting of denaturation at 94°C for 20 s, annealing and extension at 65°C for 50 s, followed by 20 three-temperature cycles with a decreased annealing temperature of 61°C and an extension at 72°C for 30 s. For visualization, 8 µl of the amplification product were run on a 2% agarose gel prestained with ethidium bromide (0.2 µg/ml).

### d) Sequencing of PCR products

Sequencing of PCR products was performed using Amplitaq^{™} DNA Polymerase FS cycle sequencing chemistry and base specific fluorescence-labeled dideoxynucleotide termination reagents (dye terminators).

The PCR products were purified by streptavidin-coated paramagnetic Dynabeads M-280 Streptavidin (Dynal, Oslo, Norway). The biotinylated product was attached to the beads by incubating 40 µl of the PCR product for 15 min with 400 µg Dynabeads at 40°C. The beads had been washed previously and resuspended in 40 µl binding and washing buffer (B&W) (2 x buffer: 2 M NaCl, 10 mM Tris-HCL pH 7.5, 1 mM EDTA). After incubation, the PCR products were washed twice in B&W buffer and resuspended in 20 µl dH₂O. For the sequencing reaction, 1 µl of the purified PCR product was mixed with 3 pmol sequencing primer and 8 µl Terminator Ready Reaction Mix containing fluorescence labeled ddNTPs and alpha-thio dNTPs (Applied Biosystems, Foster City, CA). The final reaction volume was adjusted to 20 µl with dH₂O. Cycle sequencing was carried out in a GeneAmp PCR System 9600 for 25 cycles. After an initial denaturation step at 96°C for 10 s, the temperature was rapidly decreased to 50°C and held for 5 s, followed by the extension step at 60°C for 4 min. For the subsequent removal of dye-labeled nucleotides, Sephadex G-50 columns DNA Grade F (MicroSpin G50 columns, Pharmacia Biotech, Uppsala, Sweden) were used. The pellet was dried in a vacuum centrifuge and resuspended in 4 µl loading buffer containing deionized formamide/25 mM EDTA; pH 8.0 (5:1) and Blue dextran (50 mg/ml solution). Samples were heated for 2 min at 90°C to denature and 1.5 µl were loaded on a 0.2 mm thick 5% polyacrylamide-7M urea gel. Electrophoresis was run at constant 48 watt for 8 h on an ABI 377 automatic DNA sequencer (Applied Biosystems, software version 2.1.1).

### Example 2

### Alignment of the 1st intron sequences

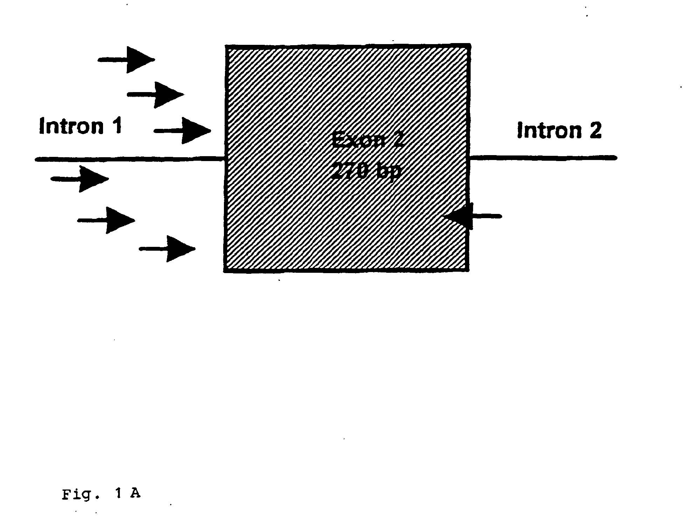

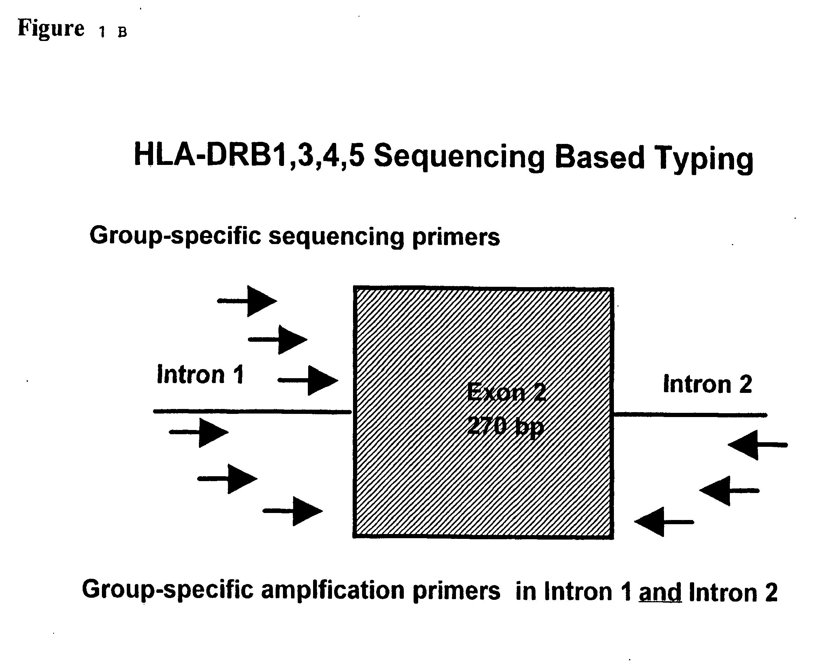

All cell lines and clinical samples were sequenced for the investigated regions in both directions. Each of the serological defined groups was analysed several times in unrelated samples as indicated in Figure 1 (part I). Rare alleles could only be sequenced once or twice due to their limited availability. Figure 1 shows from the 6400 bp sized intron 1 the sequence alignment of the 486 bp 3' end fragment directly upstream of exon 2 and the deduced consensus of the coding HLA-DRB loci. Nucleotide agreement with the consensus is indicated by a hyphen (-), asterix (*) are introduced to achieve maximum alignment. The numbers above the sequences refer to the nucleotide positions and start at the 5' end of the sequenced fragment of the 6400 bp intron 1.

The intron sequences turned out to be highly polymorphic. Besides extensive homologies, numerous locus- and group-specific sites including stretches of nucleotide deletions could be identified. Most of the polymorphic motifs or deletions are related to serological families. This lineage specificity is the most remarkable feature of the intron sequences. Beyond that, there are close relationships between the HLA-DRB1 alleles belonging to the same haplotype group, i.e. the alleles of the DR51, DR52 and DR53 associated groups. The highest sequence similarity has been found in the DR51 associated alleles of DRB1*15 and 16. In the DR53 associated groups DRB1*0701 and DRB1*0901 are closer to each other than one of them to DRB1*04. The DR52 associated alleles exhibit strong homologies with the exception of DRB1*12, which exhibits numerous individual motifs. The DRB1*11 and DRB1*13 alleles are closer to each other than one of them to DRB1*14 alleles.

With two exceptions, for each serological group at least one unique sequence motif not shared by any other group has been identified. The DRB1*11 alleles do not exhibit any motif which is more specific than the common DR52 associated DRB1 group motifs. The individual motifs in DRB1*16 are shared with DRB1*04 alleles at position 141 and with DRB5 alleles at position 181.

The sequences turned out to have an extensive stability within each lineage. Individual sequence motifs of alleles belonging to the same serologically defined DRB1 group are the exceptions to the rule. Allelic single nucleotide differences have been found in the DRB1*15 group between positions 211-220. In the DRB1*13 group the DRB1*1302 bears the consensus sequence at position 54 and a unique motif at position 328, the DRB1*1302 and 1305 are lacking the DR13 specific motif at position 370. In the DRB1*08 group the DRB1*0803 has a unique motif at position 342.

The DRB3 alleles follow the same rule with complete intron sequence homologies within the serologically defined DR52a, b and c groups, but clearly unique differences between them. Within the DRB4 group some individual motifs could be identified in the DRB4*0101 allele (positions 2, 27, 61 and 412). Among the two DRB5 alleles sequenced so far (DRB5*0101 and 0202) for their 1st intron four differences could be identified (positions 49, 287, 305 and 438).

Figure 3 shows different primers which were perferably used for the amplification and sequencing of the specimens to be classified. Figure 4 shows a compilation of different primers and their use in determining the different HLA Class II genes or HLA-DRB genes.

### Example 3

For typing the group of a patient in the first step the DNA is isolated from a blood sample. The isolated DNA is checked in the first step with the low-resolution typing system containing 24 primer mixes (as shown in Figure 5). This method can be designated as "PCR-SSP". The abbreviation "SSP" means sequence specific primer since the primer is specific for certain sequence motifs and only if those sequences are present in the DNA obtained from the patient an amplification of the DNA is obtained. The information obtained by this type of amplification is therefore simply obtained from the presence or absence of an amplification product which may be obtained by performing the different amplifications in parallel.

In the present example an amplification was obtained by using the primer mix 1.1, 15 and 24. This means that the patient shows the serology equivalent type HLA-DR1, 15; DR51.

In the next step the alleles of DRB1^{*}01, DRB1^{*}15 and DRB5^{*} have to be determined. Therefore, suitable primer mixes as listed in Figure 4 are used in order to prepare products suitable for sequencing. In the present example the primer mixes number 1, 2 and 13 were used. After amplification the sequencing primers as given in Figure 4 were used.

By using this procedure the following type of the patient could be obtained: HLA-DRB1^{*}0102,1501; DRB5^{*}0202.

A typing as described in the present example has the advantage that the cis/trans linkage of the mutations in exon 2 can be defined. Many different alleles of the DRB gene are not characterized by specific sequence motifs but by a different compilation selected from a pool of sequence motifs. If the single alleles are not amplified in single batches but both alleles together (in the present example DRB1^{*}0102 and 1501) different heterozygous positions are obtained by sequencing. From the results it cannot be concluded which positions are linked on one chromosome and which position belongs to the other chromosome. In these cases the typing is done by using suitable software which checks all possible cis/trans linkages against the most recent sequence databank. In case new alleles are discovered later it may happen that the type is no longer correct since the future sequence databank comprising further alleles may now also find further possible cis/trans linkages. This is a very common problem of known sequencing techniques and also the problem of the "PCR-SSO". The term "SSO" means hybridizing with sequence-specific oligonucleotides. In this method the PCR product obtained after a gene locus-specific amplification comprising usually at least two alleles is tested with many different oligonucleotides and on the basis of the so obtained sequence motifs and based on the sequence databank a typing is deduced.

## Claims

1. A method of determining the HLA Class II group type which is part of the HLA-DRB1, HLA-DRB3, HLA-DRB4 or HLA-DRB5 locus of a subject comprising the following steps:
(i) combining a group-specific primer pair with a target DNA sample from the subject under conditions such that primer-based amplification of the target DNA may occur; and
(ii) determining whether a nucleic acid product is produced by the amplification;
wherein the ability of the primer pair to produce a nucleic acid product is associated with a particular HLA group type **characterized** is that the sense primer is located within intron 1 and the antisense primer is located within intron 2.

2. The method of claim 1, further comprising the step of
(iii) determining the nucleic acid sequence of the nucleic acid product of step (ii).

3. The method of claim 1, wherein the primer pair comprises one or more oligonucleotide primers selected from the group consisting of SEQ ID NOs: 2 - 16, 18 - 23 and 70 - 95.

4. A method of determining the HLA Class II group which is part of the HLA-DRB1, HLA-DRB3, HLA-DRB4 or HLA-DRB5 locus or allele type of a subject comprising the following steps:
(i) combining a group-specific intron region primer pair with a target DNA sample from the subject under conditions such that primer-based amplification of the target DNA may occur, wherein the sense primer is located in intron 1 and the antisense primer is located within intron 2;
(ii) hybridizing the product of amplification with at least one sequence-specific oligonucleotide probe;
(iii) obtaining a partial sequence of the product of amplification by determining which sequence-specific oligonucleotide has hybridized;
(iv) identifying from the list of known HLA Class II sequences the group or allele which contain(s) the partial sequence obtained in (iii).

5. A method of determining the HLA Class II group which is part of the HLA-DRB 1, HLA-DRB3, HLA-DRB4 or HLA-DRB5 locus or allele type of a subject comprising the following steps:
(i) combining loci specific intron region primer pair with a target DNA sample from the subject under conditions such that the primer-based amplification of the target DNA may occurv wherein the sense primer is located in intron 1 and the antisense primer is located within intron 2;
(ii) hybridizing the product of amplification with at least one group-specific oligonucleotide probe.

## Patentansprüche

1. Verfahren zum Bestimmen des Typus der HLA-Klasse-II-Gruppe, die ein Teil des HLA-DRB1-, HLA-DRB3-, HLA-DRB4- oder HLA-DRB5-Locus ist, eines Individuums, umfassend die folgenden Schritte:
(i) Kombinieren eines Gruppen-spezifischen Primerpaares mit einer Target-DNA-Probe von dem Individuum unter Bedingungen, bei denen Primer-basierte Amplifikation der Target-DNA stattfinden kann; und
(ii) Bestimmen, ob durch die Amplifikation ein Nukleinsäureprodukt erzeugt wurde;
wobei die Fähigkeit des Primerpaares zur Erzeugung eines Nukleinsäureproduktes mit einem bestimmten Typus von HLA-Gruppe assoziiert ist, **dadurch gekennzeichnet, dass** der Sense-Primer in Intron 1 lokalisiert ist und der Antisense-Primer in Intron 2 lokalisiert ist.

2. Verfahren nach Anspruch 1, ferner umfassend den Schritt
(iii) des Bestimmens der Nukleinsäuresequenz des Nukleinsäureproduktes aus Schritt (ii).

3. Verfahren nach Anspruch 1, wobei das Primerpaar einen oder mehrere Oligonucleotidprimer umfasst, ausgewählt aus der Gruppe, bestehend aus den SEQ ID Nr.: 2 - 16, 18 - 23 und 70 - 95.

4. Verfahren zum Bestimmen der HLA-Klasse-II-Gruppe, die ein Teil des HLA-DRB1-, HLA-DRB3-, HLA-DRB4- oder HLA-DRB5-Locus ist, oder eines Alleltyps eines Individuums, umfassend die folgenden Schritte:
(i) Kombinieren eines Gruppen-spezifischen Intronregion-Primerpaares mit einer Target-DNA-Probe von dem Individuum unter Bedingungen, bei denen Primer-basierte Amplifikation der Target-DNA stattfinden kann, wobei der Sense-Primer in Intron 1 lokalisiert ist und der Antisense-Primer in Intron 2 lokalisiert ist;
(ii) Hybridisieren des Amplifikationsproduktes mit mindestens einer Sequenzspezifischen Oligonucleotidsonde;
(iii) Erhalten einer Teilsequenz des Amplifikationsproduktes durch Bestimmen, welches Sequenz-spezifische Oligonucleotid hybridisiert hat;
(iv) Identifizieren der Gruppe oder des Allels, welche(s) die in (iii) enthaltene Teilsequenz enthält, aus der Liste bekannter HLA-Klasse-II-Sequenzen.

5. Verfahren zum Bestimmen der HLA-Klasse-II-Gruppe, die ein Teil des HLA-DRB1-, HLA-DRB3-, HLA-DRB4- oder HLA-DRB5-Locus ist, oder eines Alleltyps eines Individuums, umfassend die folgenden Schritte:
(i) Kombinieren eines Loci-spezifischen Intronregion-Primerpaares mit einer Target-DNA-Probe von dem Individuum unter Bedingungen, bei denen Primer-basierte Amplifikation der Target-DNA stattfinden kann, wobei der Sense-Primer in Intron 1 lokalisiert ist und der Antisense-Primer in Intron 2 lokalisiert ist;
(ii) Hybridisieren des Amplifikationsproduktes mit mindestens einer Gruppenspezifischen Oligonucleotidsonde.

## Revendications

1. Procédé de détermination du type de groupe de HLA Classe II qui fait partie du locus HLA-DRB-1, HLA-DRB3, HLA-DRB4 ou HLA-DRB5 d'un sujet comprenant les étapes suivantes :
(i) combiner une paire d'amorces spécifique au groupe avec un échantillon d'ADN cible provenant du sujet dans des conditions telle qu'une amplification basée sur l'amorce de l'ADN cible peut se produire ; et
(ii) déterminer si un produit d'acide nucléique est produit par l'amplification ;
dans lequel la capacité de la paire d'amorces à produire un produit d'acide nucléique est associée à un type de groupe HLA particulier **caractérisé en ce que** l'amorce sens est située au sein de l'intron 1 et l'amorce antisens est située au sein de l'intron 2.

2. Procédé selon la revendication 1, comprenant en outre l'étape consistant à
(iii) déterminer la séquence d'acides nucléiques du produit d'acide nucléique de l'étape (ii).

3. Procédé selon la revendication 1, dans lequel la paire d'amorces comprend une ou plusieurs amorces d'oligonucléotide choisies dans le groupe constitué de SEQ ID No. : 2 à 16, 18 à 23 et 70 à 95.

4. Procédé de détermination du groupe de HLA Classe II qui fait partie du locus HLA-DRB-1, HLA-DRB3, HLA-DRB4 ou HLA-DRB5 ou un type allèle d'un sujet comprenant les étapes suivantes :
(i) combiner une paire d'amorces de région d'intron spécifique au groupe avec un échantillon d'ADN cible provenant du sujet dans des conditions telles qu'une amplification basée sur l'amorce de l'ADN cible peut se produire, dans lequel l'amorce sens est située dans l'intron 1 et l'amorce antisens est située dans l'intron 2 ;
(ii) hybrider le produit d'amplification avec au moins une sonde d'oligonucléotide spécifique à la séquence ;
(iii) obtenir une séquence partielle du produit d'amplification en déterminant quel oligonucléotide spécifique à la séquence s'est hybridé ;
(iv) identifier dans la liste des séquences connues de HLA Classe II le groupe ou l'allèle qui contien(nen)t la séquence partielle obtenue en (iii).

5. Procédé de détermination du groupe de HLA Classe II qui fait partie du locus HLA-DRB-1, HLA-DRB3, HLA-DRB4 ou HLA-DRB5 ou un type allèle d'un sujet comprenant les étapes suivantes :
(i) combiner une paire d'amorces de région d'intron spécifique aux locus avec un échantillon d'ADN cible provenant du sujet dans des conditions telles qu'une amplification basée sur l'amorce de l'ADN cible peut se produire, dans lequel l'amorce sens est située dans l'intron 1 et l'amorce antisens est située dans l'intron 2 ;
(ii) hybrider le produit d'amplification avec au moins une sonde d'oligonucléotide spécifique au groupe ;


### SEQUENCE LISTING

(1) GENERAL INFORMATION:
   APPLICANT:
   (A) NAME: Biotest AG
      (B) STREET: Landsteinerstr. 5
      (C) CITY: Dreieich
      (E) COUNTRY: Germany
      (F) POSTAL CODE (ZIP): 63303
   TITLE OF INVENTION:
      A method for determining the Histocompatibility Locus Antigen Class II
   NUMBER OF SEQUENCES: 95
   COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PADAT Sequenzmodul, Version 1.0
(2) INFORMATION FOR SEQ ID NO: 1:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 1:
      AGCGGAGTGG AGAGGTCTG 19
(2) INFORMATION FOR SEQ ID NO: 2:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 2:
      TCCCAGGAGG AGGCGGGA 18
(2) INFORMATION FOR SEQ ID NO: 3:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 3:
      AGCGCCCGCA CCCCGCT 17
(2) INFORMATION FOR SEQ ID NO: 4:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 4:
      AAAAGCCTGG GGATCAGAAG T 21
(2) INFORMATION FOR SEQ ID NO: 5:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 5:
      GCCCCTGGGC TGCGTGTT 18
(2) INFORMATION FOR SEQ ID NO: 6:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 6:
      TGGTGGGCGT TGCGGCG 17
(2) INFORMATION FOR SEQ ID NO: 7:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 7:
      AGTGTCTTCT CAGGAGGCCA 20
(2) INFORMATION FOR SEQ ID NO: 8:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA (xi) SEQUENCE DESCRIPTION: SEQ ID NO: 8:
      TGGGCGTTTG CGGCGGGC 18
(2) INFORMATION FOR SEQ ID NO: 9:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 9:
      GAGCCCCTGG GCTGCAGA 18
(2) INFORMATION FOR SEQ ID NO: 10:
   (i) SEQUENCE CHARACTERISTICS:
      (A) LENGTH: 17 base pairs
      (B) TYPE: amino acid
      (C) STRANDEDNESS: single
      (D) TOPOLOGY: linear (ii) MOLECULE TYPE: Genomic DNA (xi) SEQUENCE DESCRIPTION: SEQ ID NO: 10:
         GCGGGTGGGG CCGGGTC 17
(2) INFORMATION FOR SEQ ID NO: 11:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 11:
      AGCGCAGGCC AGGCACAAA 19
(2) INFORMATION FOR SEQ ID NO: 12:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 12:
      GGATGGTGGC GTCTCTGTT 19
(2) INFORMATION FOR SEQ ID NO: 13:
   (i) SEQUENCE CHARACTERISTICS:
      (A) LENGTH: 17 base pairs
      (B) TYPE: amino acid
      (C) STRANDEDNESS: single
      (D) TOPOLOGY: linear (ii) MOLECULE TYPE: Genomic DNA (xi) SEQUENCE DESCRIPTION: SEQ ID NO: 13:
         GGCGTTGCGG GTGGGCG 17
(2) INFORMATION FOR SEQ ID NO: 14:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 14:
      CCCCGTTCGC CTCAGGAAA 19
(2) INFORMATION FOR SEQ ID NO: 15:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 15:
      CCTCAGGAAG ACTGAGGAC 19
(2) INFORMATION FOR SEQ ID NO: 16:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 16:
      CCAGAATAGG CTGGAGGCG 19
(2) INFORMATION FOR SEQ ID NO: 17:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 17:
      GCCGCTGCAC TGTGAAGCTC 20
(2) INFORMATION FOR SEQ ID NO: 18:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 18:
      GCCCGTGTGA CCGGATCC 18
(2) INFORMATION FOR SEQ ID NO: 19:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 19:
      CCGCCCTGTG ACCGGATG 18
(2) INFORMATION FOR SEQ ID NO: 20:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 20:
      GGATCGTTCG TGTCCCCAC 19
(2) INFORMATION FOR SEQ ID NO: 21:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 21:
      CCGGAGGCCG CTTCTGTA 18
(2) INFORMATION FOR SEQ ID NO: 22:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA (xi) SEQUENCE DESCRIPTION: SEQ ID NO: 22:
      CGCCCCTGTG ACCGGATC 18
(2) INFORMATION FOR SEQ ID NO: 23:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 23:
      GCTGTCAGTG TCTTCTCAGG 20
(2) INFORMATION FOR SEQ ID NO: 24:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 24:
      CTTGTGGCAG CTTAAGTTTG AA 22
(2) INFORMATION FOR SEQ ID NO: 25:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 25:
      CCTGTGGCAG CCTAAGAGG 19
(2) INFORMATION FOR SEQ ID NO: 26:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 26:
      AGTACTCTAC GGGTGAGTGT T 21
(2) INFORMATION FOR SEQ ID NO: 27:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 27:
      GAGTACTCTA CGGGTGAGTG TT 22
(2) INFORMATION FOR SEQ ID NO: 28:
   (i) SEQUENCE CHARACTERISTICS:
      (A) LENGTH: 18 base pairs
      (B) TYPE: amino acid
      (C) STRANDEDNESS: single
      (D) TOPOLOGY: linear (ii) MOLECULE TYPE: Genomic DNA (xi) SEQUENCE DESCRIPTION: SEQ ID NO: 28:
         GACGGAGCGG GTGCGGTA 18
(2) INFORMATION FOR SEQ ID NO: 29:
   (i) SEQUENCE CHARACTERISTICS:
      (A) LENGTH: 18 base pairs
      (B) TYPE: amino acid
      (C) STRANDEDNESS: single
      (D) TOPOLOGY: linear (ii) MOLECULE TYPE: Genomic DNA (xi) SEQUENCE DESCRIPTION: SEQ ID NO: 29:
         CGGGTGCGGT TCCTGGAG 18
(2) INFORMATION FOR SEQ ID NO: 30:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 30:
      GTTTCTTGGA GCAGGTTAAA CA 22
(2) INFORMATION FOR SEQ ID NO: 31:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 31: GGTGCAGTTC CTGGAAAGAC T 21
(2) INFORMATION FOR SEQ ID NO: 32:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 32:
      GTTTCTTGGA GTACTCTACG TC 22
(2) INFORMATION FOR SEQ ID NO: 33:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 33:
      CATAACCAGG AGGAGCTCC 19
(2) INFORMATION FOR SEQ ID NO: 34:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 34:
      GGACAGATAC TTCCATAACC AG 22
(2) INFORMATION FOR SEQ ID NO: 35:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 35:
      GGAGAGATAC TTCCATAACC AG 22
(2) INFORMATION FOR SEQ ID NO: 36:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 36:
      CTTCCATAAC CAGGAGGAGT T 21
(2) INFORMATION FOR SEQ ID NO: 37:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 37:
      ACAGCACGTT TCTTGGAGCT 20
(2) INFORMATION FOR SEQ ID NO: 38:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 38:
      GTGCGGTTCC TGCACAGAG 19
(2) INFORMATION FOR SEQ ID NO: 39:
   (i) SEQUENCE CHARACTERISTICS:
      (A) LENGTH: 23 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 39:
      GTTCCTGGAC AGATACTTCT ATC 23
(2) INFORMATION FOR SEQ ID NO: 40:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 40:
      GGTTTCTTGG AGTACTCTAC GT 22
(2) INFORMATION FOR SEQ ID NO: 41:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 41:
      GATCGTTCGT GTCCCCACAG 20
(2) INFORMATION FOR SEQ ID NO: 42:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 42:
      CACAGCACGT TTCTTGGAGG 20
(2) INFORMATION FOR SEQ ID NO: 43:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 43:
      ACGGAGCGGG TGCGGTTC 18
(2) INFORMATION FOR SEQ ID NO: 44:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 44:
      CCGCCTCTGC TCCAGGAG 18
(2) INFORMATION FOR SEQ ID NO: 45:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 45:
      CCCGCTCGTC TTCCAGGAT 19
(2) INFORMATION FOR SEQ ID NO: 46:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 46:
      TCCACCGCGG CCCGCGC 17
(2) INFORMATION FOR SEQ ID NO: 47:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 47:
      TAGGTGTCCA CCGCGGCG 18
(2) INFORMATION FOR SEQ ID NO: 48:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 48:
      TGCAGTAGGT GTCCACCAG 19
(2) INFORMATION FOR SEQ ID NO: 49:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 49:
      TCCACCGCGG CCCGCTC 17
(2) INFORMATION FOR SEQ ID NO: 50:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 50:
      TCTGCAGTAG TTGTCCACCC 20
(2) INFORMATION FOR SEQ ID NO: 51:
   (i) SEQUENCE CHARACTERISTICS:
      (A) LENGTH: 23 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 51:
      ACCCCGTAGT TGTGTCTGCA CAC 23
(2) INFORMATION FOR SEQ ID NO: 52:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 52:
      CTGTTCCAGT ACTCGGCGCT 20
(2) INFORMATION FOR SEQ ID NO: 53:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 53:
      CTGCACTGTG AAGCTCTCAC 20
(2) INFORMATION FOR SEQ ID NO: 54:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 54:
      CTGGCTGTTC CAGTACTCCT 20
(2) INFORMATION FOR SEQ ID NO: 55:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 55:
      CTGCACTGTG AAGCTCTCCA 20
(2) INFORMATION FOR SEQ ID NO: 56:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 56:
      CCCGCCTGTC TTCCAGGAT 19
(2) INFORMATION FOR SEQ ID NO: 57:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 57:
      CCGCGCCTGC TCCAGGAT 18
(2) INFORMATION FOR SEQ ID NO: 58:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 58:
      CCCGCCTGTC TTCCAGGAA 19
(2) INFORMATION FOR SEQ ID NO: 59:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 59:
      CCCGCTCGTC TTCCAGGAA 19
(2) INFORMATION FOR SEQ ID NO: 60:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 60:
      CTCGCTGTTC CAGTACTCGG 20
(2) INFORMATION FOR SEQ ID NO: 61:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 61:
      TCTCGCTGTT CCAGTACTCA G 21
(2) INFORMATION FOR SEQ ID NO: 62:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 62:
      CACCTCGGCC CGCCTCC 17
(2) INFORMATION FOR SEQ ID NO: 63:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 63:
      CACCGCGGCC CGCCTCT 17
(2) INFORMATION FOR SEQ ID NO: 64:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 64:
      GTCCACCGCG GCGCGCTT 18
(2) INFORMATION FOR SEQ ID NO: 65:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 65:
      CTGCAGTAGG TGTCCACCAG 20
(2) INFORMATION FOR SEQ ID NO: 66:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 66:
      CTGTTCCAGT GCTCCGCAG 19
(2) INFORMATION FOR SEQ ID NO: 67:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 67:
      CGTAGTTGTG TCTGCAGTAA T 21
(2) INFORMATION FOR SEQ ID NO: 68:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 68:
      CTCCACAACC CCGTAGTTGT A 21
(2) INFORMATION FOR SEQ ID NO: 69:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 69:
      CCGCTGCACT GTGAAGCTCT 20
(2) INFORMATION FOR SEQ ID NO: 70:
   (i) SEQUENCE CHARACTERISTICS:
      (A) LENGTH: 18 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 70:
      TCCCAGTGCC CGCACCCT 18
(2) INFORMATION FOR SEQ ID NO: 71:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 71:
      GGTGGGTGCT GTTGAAGGT 19
(2) INFORMATION FOR SEQ ID NO: 72:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 72:
      AGCACTAAGG AAGGGTTCAG 20
(2) INFORMATION FOR SEQ ID NO: 73:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 73:
      GGGATCAGAG GTAGTTTTTC CA 22
(2) INFORMATION FOR SEQ ID NO: 74:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 74:
      AACAGGCTGG AGGTAGGGAC 20
(2) INFORMATION FOR SEQ ID NO: 75:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
      (xi) SEQUENCE DESCRIPTION: SEQ ID NO: 75: TGGTGGGCGT TGCGGCG 17
(2) INFORMATION FOR SEQ ID NO: 76:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 76:
      GTGGGCGTTG CGGCGGC 17
(2) INFORMATION FOR SEQ ID NO: 77:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 77:
      GTTTTCCCGC CTGGTCCCT 19
(2) INFORMATION FOR SEQ ID NO: 78:
   (i) SEQUENCE CHARACTERISTICS:
      (A) LENGTH: 19 base pairs
      (B) TYPE: amino acid
      (C) STRANDEDNESS: single.
      (D) TOPOLOGY: linear
   (ii) MOLECULE TYPE: Genomic DNA
   (xi) SEQUENCE DESCRIPTION: SEQ ID NO: 78:
      CGGCGTCGCT GTCAGTGTT 19
(2) INFORMATION FOR SEQ ID NO: 79:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 79:
      AGCGCAGGCC AGGCACAAA 19
(2) INFORMATION FOR SEQ ID NO: 80:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 80:
      CAGTTAAGGT TCCAGTGCCA 20
(2) INFORMATION FOR SEQ ID NO: 81:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 81:
      TTTCAGGTGA AGACTCCCAG A 21
(2) INFORMATION FOR SEQ ID NO: 82:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 82:
      GATGAGAGAA GGAGCAGAGA T 21
(2) INFORMATION FOR SEQ ID NO: 83:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 83:
      GGCGTTGCGG GTGGGCG 17
(2) INFORMATION FOR SEQ ID NO: 84:
   (i) SEQUENCE CHARACTERISTICS:
      (A) LENGTH: 22 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 84:
      CACAAGGTCA TCACTAAGAA AG 22
(2) INFORMATION FOR SEQ ID NO: 85:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 85:
      AGCACTAAGG AAGGGTTCAC 20
(2) INFORMATION FOR SEQ ID NO: 86:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 86:
      GTTTTCCCGC CTGGTCCCC 19
(2) INFORMATION FOR SEQ ID NO: 87:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 87:
      ACACACTCAG ATTCTCCGCT T 21
(2) INFORMATION FOR SEQ ID NO: 88:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 88:
      ACACACACAC TCAGATTCTC C 21
(2) INFORMATION FOR SEQ ID NO: 89:
   (i) SEQUENCE CHARACTERISTICS:
      (A) LENGTH: 20 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 89:
      TCAGATTCCC AGCTCGGAGA 20
(2) INFORMATION FOR SEQ ID NO: 90:
   (i) SEQUENCE CHARACTERISTICS:
      (A) LENGTH: 17 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 90:
      CAGGCCCCGC CCCCCGA 17
(2) INFORMATION FOR SEQ ID NO: 91:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 91:
      ACACACACAC TCAGATTCCC A 21
(2) INFORMATION FOR SEQ ID NO: 92:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 92:
      ACACACATAC ACAGATTCCC C 21
(2) INFORMATION FOR SEQ ID NO: 93:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 93:
      ACACACAGAG TCAGATTCCC A 21
(2) INFORMATION FOR SEQ ID NO: 94:
   (i) SEQUENCE CHARACTERISTICS:
      (A) LENGTH: 21 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 94:
      CACACACACA CTCTCAGATT T 21
(2) INFORMATION FOR SEQ ID NO: 95:
   (i) SEQUENCE CHARACTERISTICS:
      (A) LENGTH: 19 base pairs
      (B) TYPE: amino acid
      (C) STRANDEDNESS: single
      (D) TOPOLOGY: linear
   (ii) MOLECULE TYPE: Genomic DNA
   (xi) SEQUENCE DESCRIPTION: SEQ ID NO: 95:
      TGTCACCTCC CCACAGAGT 19